(19) Europäisches Patentamt
European Patent Office
Office européen des brevets

(11) **EP 3 856 024 B1**

(12) **EUROPEAN PATENT SPECIFICATION**

(45) Date of publication and mention
of the grant of the patent:
**02.07.2025 Bulletin 2025/27**

(21) Application number: **19866489.8**

(22) Date of filing: **20.09.2019**

(51) International Patent Classification (IPC):
***A61B 5/053*** (2021.01)      ***A61B 5/0537*** (2021.01)

(52) Cooperative Patent Classification (CPC):
**A61B 5/0537; A61B 5/7221;** A61B 5/0205

(86) International application number:
**PCT/AU2019/051006**

(87) International publication number:
**WO 2020/061619 (02.04.2020 Gazette 2020/14)**

(54) **EVALUATING IMPEDANCE MEASUREMENTS**

AUSWERTUNG VON IMPEDANZMESSUNGEN

ANALYSE DE MESURES D'IMPÉDANCE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.09.2018   US 201862737721 P
03.05.2019   US 201962842804 P**

(43) Date of publication of application:
**04.08.2021   Bulletin 2021/31**

(73) Proprietor: **Impedimed Limited
Pinkenba, QLD 4008 (AU)**

(72) Inventors:
• **GAW, Richelle Leanne
Wakerley, Queensland 4154 (AU)**
• **TERRONES, Benjamin Sanchez
Brookline, Massachusetts 02445 (US)**

(74) Representative: **Appleyard Lees IP LLP
15 Clare Road
Halifax HX1 2HY (GB)**

(56) References cited:
**US-A1- 2010 168 530      US-A1- 2010 168 530
US-A1- 2010 268 110      US-A1- 2012 323 237
US-A1- 2017 325 711      US-A1- 2017 325 711
US-A1- 2018 184 941      US-B1- 9 949 663**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Background of the Invention

The present invention relates to a method and system for performing impedance measurements and in particular to evaluating the impedance measurements, for example to confirm the impedance measurement is valid.

## Description of the Prior Art

The reference in this specification to any prior publication (or information derived from it), or to any matter which is known, is not, and should not be taken as an acknowledgment or admission or any form of suggestion that the prior publication (or information derived from it) or known matter forms part of the common general knowledge in the field of endeavour to which this specification relates.

One existing technique for determining biological indicators relating to a subject, such as cardiac function, body composition, and other health status indicators, such as the presence of oedema, involves the use of bioelectrical impedance. This process typically involves using a measuring device to measure the electrical impedance of a subject's body using a series of electrodes placed on the skin surface, although other techniques could be used. Changes in electrical impedance are used to determine parameters, such as changes in fluid levels, associated with the cardiac cycle, oedema, or the like.

Impedance measuring apparatus is sometimes sensitive to external factors, including stray capacitances between the subject and the local environment and the measurement apparatus, variations in electrode/tissue interface impedances, also known as electrode impedances, as well as stray capacitances and inductive coupling between the leads used to connect the measuring device to the electrodes, meaning measurements performed by an impedance measuring device are not always accurate.

Measurements are typically evaluated manually by having user's view a visual representation of measured impedance values to see if these "look correct". Such a subjective assessment is difficult for inexperienced users to perform, meaning accurate measurements may be discarded, whilst inaccurate measurements are used, which can in turn can lead to incorrect calculation of biological indicators.

US20170325711A1 discloses a method and apparatus for testing the plausibility of electric impedance measurement values. The measurement values are determined during the measurement of the bioimpedance of a person. US20180184941A1 discloses an apparatus for analysis of body composition and hydration status by detecting resistance of the human subject at zero and infinite frequency including a method for measuring indirectly extracellular water mass, intracellular water mass, lean body mass, and body fat mass. US20100168530A1 discloses a method for determining biological indicators, the method including, in a processing system causing at least one radiation attenuation measurement to be performed and determining at least one first biological indicator using determined radiation attenuation. US20100268110A1 discloses a method of monitoring mucosal injury due to hypoperfusion and ischemia in the critically ill.

## Summary of the Present Invention

The invention is set forth in the claims.

In one broad form an aspect of the present invention seeks to provide a system for performing a bioimpedance measurement on a biological subject as provided in claim 1.

In one broad form an aspect of the present invention seeks to provide a method for use in evaluating a bioimpedance measurement performed on a biological subject as provided in claim 15.

In one embodiment the impedance curve is a circular segment.

In embodiments the evaluation of the impedance measurements is used to categorise the impedance measurement; and, derive an indicator indicative a measurement categorisation.

In one embodiment the impedance measurement is categorised as being at least one of: bad; questionable; and, acceptable.

In one embodiment the one or more processing devices: use the deviation to calculate an estimated error in impedance parameter values, the impedance parameter values being derived from the impedance curve; and, use the estimated error to evaluate the impedance measurements.

In one embodiment the one or more processing devices calculate a respective estimated error for each of a number of impedance parameter values.

In one embodiment the one or more processing devices: compare the estimated error to one or more thresholds; and, evaluate the impedance measurement based on results of the comparison.

In one embodiment one or more processing devices: calculate curve coefficients using the curve fitting algorithm; and, determine the deviation using the curve coefficients and the impedance values.

**In** one embodiment the curve fitting algorithm uses a method of least squares.

**In** one embodiment the one or more processing devices calculate an estimated error by propagating the deviation to the impedance parameter values.

**In** one embodiment the deviation is based on at least one of: a variance; and, a co-variance.

In one embodiment the one or more processing devices: determine the deviation by calculating a co-variance matrix indicative of variances and co-variances associated with the impedance curve and the impedance values; and, use the co-variance matrix to estimate the error.

In one embodiment the co-variance matrix is generated based on a mean square error of the impedance curve and the impedance values.

In one embodiment the one or more processing devices calculate the co-variance matrix using the equation:

$$\hat{\sigma}_{\hat{x}}^2 = \hat{\sigma}_{\hat{y}}^2 (A^T A)^{-1}$$ where: A is a matrix of measured reactance values X and resistance values R of the form

$$A = \begin{bmatrix} 1 & R & X \end{bmatrix}$$ $\hat{\sigma}_{\hat{y}}^2$ is the mean square error of the curve fit.

In one embodiment the one or more processing devices calculate an estimated error using first order partial derivatives of a vector function.

In one embodiment the one or more processing devices calculate an estimated error using a Jacobian transform.

In one embodiment the one or more processing devices calculate an estimated error by applying a Jacobian transform to a co-variance matrix using the equation: $$\hat{\sigma}_f^2 = J_f \, \hat{\sigma}_{\hat{x}}^2 \, J_f^T$$ where: $J$ is a Jacobian transform; $J^T$ is an inverse Jacobian transform; $\hat{\sigma}_f^2$ is the error.

In one embodiment the one or more processing devices: calculate an error value associated with a number of impedance parameter values; compare each of error value to at least one respective threshold; and, perform the evaluation based on results of the comparison.

In one embodiment the number of impedance parameter values include one or more of: $R_0$ which is the theoretical impedance at a frequency of OkHz; $R_{inf}$ which is the theoretical impedance at an infinite frequency; and, $R_i$ which is the intracellular impedance.

In one embodiment the one or more processing devices calculate error values using the equations: for $R_0$:

$$\sigma_{R0} = \sqrt{J_{R0} \, \hat{\sigma}_{\hat{x}}^2 \, J_{R0}^T} \qquad \sigma_{R0}(\%) = \frac{100 \, \sigma_{R0}}{R0}$$ where: $J_{R0}$ is a Jacobian transform for $R_0$; $J^T_{R0}$ is an inverse Jacobian transform for $R_0$; and, $\hat{\sigma}_{\hat{x}}^2$ is a co-variance matrix; for $R_{inf}$ $$\sigma_{Rinf} = \sqrt{J_{Rinf} \, \hat{\sigma}_{\hat{x}}^2 \, J_{Rinf}^T} \qquad \sigma_{Rinf}(\%) = \frac{100 \, \sigma_{Rinf}}{Rinf}$$

where: $J_{Rinf}$ is a Jacobian transform for $R_{inf}$; $J^T_{Rinf}$ is an inverse Jacobian transform for $R_{inf}$; and, $\hat{\sigma}_{\hat{x}}^2$ is a co-variance matrix; and, for $R_i$ $$\sigma_{Ri} = \sqrt{J_{Ri} \, \hat{\sigma}_{\hat{x}}^2 \, J_{Ri}^T} \qquad \sigma_{Ri}(\%) = \frac{100 \, \sigma_{Ri}}{Ri}$$ where: $J_{Ri}$ is a Jacobian transform for $R_i$; $J^T_{Ri}$ is an inverse Jacobian transform for $R_i$; and, $\hat{\sigma}_{\hat{x}}^2$ is a co-variance matrix

In one embodiment the one or more processing devices evaluate the impedance measurement based on a number of negative reactance values.

In one embodiment the one or more processing devices evaluate the impedance measurement as being: in a first category if at least one of: a number of negative reactance measurements exceeds a first category reactance threshold; or at least one parameter value has an error greater than a respective first category threshold; in a second category if at least one of: a number of negative reactance measurements exceeds a second category reactance threshold; each parameter value has an error greater than a respective second category threshold; in a third category if it is not in the first or second category.

In one embodiment the one or more processing devices evaluate the impedance measurement as being: in a first category if: an $R_0$ error is greater than a first category $R_0$ threshold; in a second category if at least one of: an $R_i$ error is greater than a first category $R_i$ threshold; and, an $R_{inf}$ error is greater than a first category $R_{inf}$ threshold.

In one embodiment the one or more processing devices evaluate the measurement at least in part using one or more thresholds, and wherein the thresholds are at least one of: determined based on evaluation of previous impedance measurements performed for the subject determined based on evaluation of previous measurements impedance performed for one or more reference subjects; and, determined by applying machine learning to evaluation of previous impedance measurements performed for one or more reference subjects

In one embodiment the one or more processing devices select at least one threshold in accordance with at least one of: subject characteristics; and, an impedance analysis process being performed.

In one embodiment the one or more processing devices: evaluate the impedance measurement using at least one threshold; determine a user assessment of the impedance measurement in accordance with user input commands; and, selectively modify the at least one threshold based on the user assessment.

In one embodiment the one or more processing devices evaluate the measurement using at least one computational model embodying a relationship between different deviations and measurement validity.

In one embodiment the at least one computational model is obtained by applying machine learning to deviations and assessments of measurement validity obtained from one or more reference subjects.

In one embodiment the one or more processing devices: apply a phase correction to impedance values measured at a frequency higher than a set frequency; and, calculate the impedance curve using the phase corrected impedance values.

In one embodiment each impedance value includes a reactance value and a resistance value.

In one embodiment the one or more processing devices: determine a count of impedance values having negative reactance values; and, use the count to perform an evaluation of the impedance measurements.

In one embodiment the one or more processing devices: apply a phase correction to impedance values measured at a frequency higher than a set frequency; and, determine the count using the phase corrected impedance values.

In one embodiment the one or more processing devices evaluate the impedance measurement as being: in a first category if the count is greater than 25% of a total number of impedance values; and, in a second category if the count is greater than 14%.

In one embodiment the one or more processing devices: calculate an impedance parameter value using the impedance values; compare the impedance parameter value to a defined frequency impedance value, the defined frequency impedance value being determined from an impedance value obtained from impedance measurements performed at a defined measurement frequency; and, use a result of the comparison to perform an evaluation of the impedance measurements.

In one embodiment: the parameter value is $R_0$ which is the theoretical impedance at a frequency of OkHz; and, the defined frequency impedance value is determined from an impedance measurement performed at at least one of: greater than 1 kHz; less than 10 kHz; less than 20 kHz; less than 30 kHz; between 2 kHz and 4 kHz; and, about 3 kHz.

In one embodiment: the parameter value is $R_{inf}$ which is the theoretical impedance at an infinite frequency; and, the defined frequency impedance value is determined from an impedance measurement performed at at least one of: greater than 100 kHz; greater than 500 kHz; less than 10000 kHz; less than 5000 kHz; less than 2000 kHz; between 500 kHz and 2000 kHz; and, about 1000 kHz.

In one embodiment the parameter value is a theoretical impedance at the defined frequency.

In one embodiment the one or more processing devices evaluate the impedance measurement as being: in a first category if a magnitude of a difference between the impedance parameter value and the defined frequency impedance value is greater than a first difference threshold; and, in a second category if a magnitude of the difference between the impedance parameter value and the defined frequency impedance value is greater than a second difference threshold but less than the first difference threshold.

It will be appreciated that the broad forms of the invention and their respective features can be used in conjunction and/or independently, and reference to separate broad forms is not intended to be limiting. Furthermore, it will be appreciated that features of the method can be performed using the system or apparatus and that features of the system or apparatus can be implemented using the method.

## Brief Description of the Drawings

Examples of various embodiments of the present invention will now be described with reference to the accompanying drawings, in which: -

Figure 1A is a flow chart of an example of a method for evaluating a bioimpedance measurement performed on a biological subject;

Figure 1B is a schematic diagram of an example of a theoretical equivalent circuit for biological tissue;

Figure 1C is an example of a locus of impedance known as a Complex impedance-plot;

Figure 2 is a schematic diagram of an example of a distributed system architecture;

Figure 3A is a schematic diagram of a measuring system;

Figure 3B is a schematic diagram of a specific example of the physical construction of the impedance measuring device of Figure 3A;

Figures 3C to 3G are schematic diagrams of examples of electrode positions for use in performing impedance measurements;

Figure 4 is a schematic diagram of an example of a client device;

Figure 5 is a schematic diagram of an example of a server;

Figures 6A to 6D are a flow chart of a further example of a method for evaluating a bioimpedance measurement performed on a biological subject;

Figures 7A to 7C are graphs illustrating an example of phase correction of impedance values; and,

Figures 8A to 8C are graphs illustrating an example of phase correction calculation.

**Detailed Description of the Preferred Embodiments**

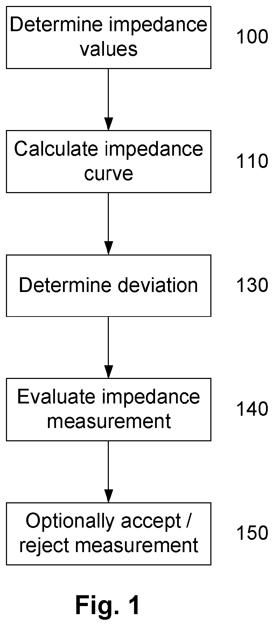

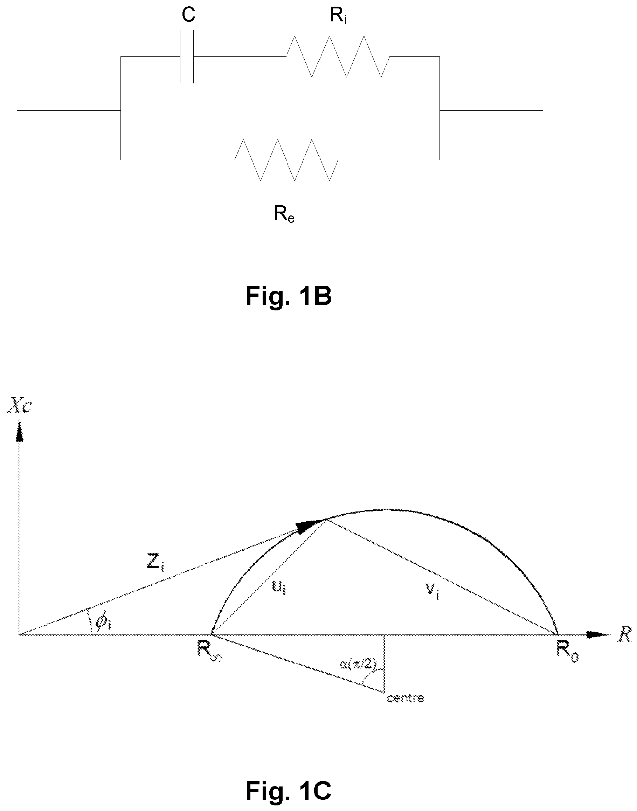

An example of a method for evaluating a bioimpedance measurement performed on a biological subject will now be described with reference to Figure 1.

For the purpose of this example, it is assumed that the impedance measurement process is being used to determine impedance parameter values, such as extrapolated values of the impedance at theoretical frequencies. These can then be used in order to determine one or more indicators indicative of a body state value, such as a fluid status, a body composition value, a disease state indicator indicative of a presence, absence, or degree of a medical condition such as heart failure, lymphedema, or the like. However, it will be appreciated that this is not essential, and that the techniques described herein could be used to evaluate bioimpedance measurements performed on biological subjects for other reasons.

For the purpose of illustration, it is also assumed that the process is performed at least in part using one or more electronic processing devices forming part of one or more processing systems, connected to one or more measuring systems, such as impedance measuring systems or the like. In one example, this is performed at least using a cloud based architecture that interfaces with measuring systems or other client devices, as will be described in more detail below. Whilst the system can use multiple processing devices, with processing performed by one or more of the devices, for the purpose of ease of illustration, the following examples will refer to a single device, but it will be appreciated that reference to a singular processing device should be understood to encompass multiple processing devices and *vice versa,* with processing being distributed between the devices as appropriate.

In one example, the measuring device includes a signal generator electrically connected to drive electrodes to apply drive signal to a user, a sensor electrically connected to sense electrodes to measure a response signal in the user and one or more processing devices that at least in part controls the signal general, receives an indication of a measured response signal from the sensor and calculates at least one measured impedance value.

For ease of explanation, the term "subject" refers to any animal or animal tissue that is being assessed, and more particularly a human, although this is not intended to be limiting and the techniques could be applied more broadly to other vertebrates, mammals or tissues.

In this example, at step 100 an impedance measurement is performed by applying electrical stimulation signals at a number of different frequencies and measuring resulting response signals in the body, allowing a number of impedance values to be determined.

In one example, the impedance values are measured over many frequencies ranging from very low frequencies (1 kHz) to higher frequencies (500 kHz or 1000 kHz), with some devices measuring impedance values at 256, or more, different frequencies within this range, although this is not essential and other configurations of measurement frequency could be used. For example, in some cases only four measurements are recorded, with these being over a range of frequencies, such as frequencies of about 25 kHz, 50 kHz, 100 kHz, and 200 kHz, or the like.

The impedance measurements can be of any form and could include whole of body or segmental impedance measurements, and examples will be described in more detail below. The impedance measurements could be performed as part of an analysis process, or alternatively could be performed by a separate measuring device, with analysis, including evaluation, being subsequently performed by the one or more processing devices, for example by retrieving results from a database, receiving the results from a measuring device, or the like.

At step 110, the impedance values are used to calculate an impedance curve, which is typically performed using a curve fitting algorithm, and in one particular example, a circle fitting algorithm, although other suitable techniques could be used.

In this regard, Figure 1B is an example of an equivalent circuit that effectively models the electrical behaviour of biological tissue. The equivalent circuit has two branches that represent current flow through extracellular fluid and intracellular fluid, respectively. The extracellular fluid component of biological impedance is represented by an extracellular resistance $R_e$, whilst the intracellular fluid component is represented by an intracellular resistance $R_i$ and a capacitance C representative of the cell membranes.

The relative magnitudes of the extracellular and intracellular components of impedance of an alternating current (AC) are frequency dependent. At zero frequency the capacitor acts as a perfect insulator and all current flows through the extracellular fluid, hence the resistance at zero frequency, $R_0$, equals the extracellular resistance $R_e$. At infinite frequency the capacitor acts as a perfect conductor and the current passes through the parallel resistive combination. The resistance at infinite frequency $R_\infty$ is given by:

$$R_\infty = \frac{R_e R_i}{R_e + R_i} \tag{1}$$

Hence the intracellular resistance is given by:

$$R_i = \frac{R_\infty R_e}{R_e - R_\infty} \tag{2}$$

Accordingly, the impedance of the equivalent circuit of Figure 1B at an angular frequency $\omega$, where $\omega=2\pi*$frequency, is given by:

$$Z = R_\infty + \frac{R_0 - R_\infty}{1+(j\omega\tau)} \tag{3}$$

where: $R_\infty$ = impedance at infinite applied frequency

$R_0$ = impedance at zero applied frequency = $R_e$ and,
$\tau$ is the time constant of the capacitive circuit.

However, the above represents an idealised situation which does not take into account the fact that the cell membrane is an imperfect capacitor. Taking this into account leads to a modified model in which:

$$Z = R_\infty + \frac{R_0 - R_\infty}{1+(j\omega\tau)^\alpha} \tag{4}$$

where: $\alpha$ has a value between 0 and I and can be thought of as an indicator of the deviation of a real system from the ideal model and represents a depression of the centre of the arc below the axis.

Each measurement provides the impedance a magnitude and impedance phase or, alternatively, resistance and reactance. Plotting the impedance values from multiple frequencies in a reactance-resistance graph often describes a semi-circular plot referred to as a complex impedance plot, and an example plot is shown in Figure 1C.

As frequency increases, the reactance increases to a peak at the characteristic frequency and then decreases while the resistance continually decreases. This results in a substantially circular locus with the centre of the circle below the x axis, as shown. In this example, $Z_i$ indicates a single impedance value, at an arbitrary frequency $f_i$, having a phase angle of $\phi_i$. The lines represented by $U_i$ and $V_i$ are chords drawn from the endpoints, $R_\infty$ and $R_0$ to $Z_i$. If the log of the ratio of all possible chords $U_i$ and $V_i$ are plotted against log frequency, this should be a straight line crossing the x axis at $f_c$ and having a slope of $(-\alpha)$.

Whilst the measured impedance can be used directly, more typically the measured impedance is used to derive an impedance parameter and in this regard, the semi-circular complex impedance plot allows the determination of a number of impedances at key theoretical values that are not directly measurable due to practical reasons.

One example, is $R_0$, which is the impedance measured at the frequency of zero kilohertz. This is also known as direct current and is not suitable for use on humans for a number of reasons. $R_0$ represents the best theoretical value of impedance in the extracellular space. At this frequency the current travels solely through the extracellular fluid compartment. Thus, the opposition (impedance) of this current is due to the extracellular fluid (ECF) alone. As can be seen from the complex impedance plot, the reactance component (caused by cellular membrane interaction with the current) of the impedance vector in this case is zero.

Another parameter is $R_{inf}$ (also referred to in literature as $R_\infty$), which is the impedance measured at the theoretical frequency of infinite kilohertz. Again, no such frequency current is achievable, but the complex impedance plot allows this to be used to extrapolate this value of impedance. This extrapolation is made possible because the frequencies measured to generate the plot allow the accurate determination of the radius of the circle. At infinite frequency, the current will not be affected by interaction with the cell membranes and will pass right through the cell membranes. Therefore, the impedance at $R_{inf}$ is the impedance due to both the extracellular and intracellular fluid, that is, the total body water. At this theoretical frequency the reactance component of impedance vector is also zero. This allows $R_\infty$ to be used with $R_0$ to derive an intracellular resistance $R_i$, as well as other impedance parameters, such as $X_c$, $Z_c$ or $\alpha$.

The nature of the curve fitting algorithm will vary depending on the preferred implementation. In one example, a regression procedure is then used to fit the measured data to the theoretical semi-circular locus, although alternatively, a circle fitting technique can be used in which three simultaneous equations representing the geometric relationships between points on a circle are solved to allow calculation of the radius (r) and the co-ordinates of the centre of the circle (i, j) as the three parameters which define the circle. It will also be appreciated that linear or non-linear methods, such as a non-linear least squares approach could be used, which can in turn simply downstream propagation of errors to the impedance parameter values, which is discussed in more detail below.

The above described equivalent circuit models the resistance as a constant value and does not therefore accurately reflect the impedance response of a subject, and in particular does not accurately model the change in orientation of the erythrocytes in the subject's blood stream, or other relaxation effects. To more successfully model the electrical conductivity of the human body, an improved CPE based model may alternatively be used. In this instance, it will be appreciated that the complex impedance plot might not define a circle, and an alternative curve could be used. Whilst the following description will focus on the use of a circular segment, it will be appreciated that this is not intended to be limiting and other curves could be used.

At step 120, the one or more processing devices determine a deviation of the impedance curve from the impedance measurements. In this regard, it will be appreciated that the impedance measurements do not generally fit exactly on the theoretical curve, with variations from the theoretical curve representing measurement inaccuracies. The deviation therefore represents variations between the measured impedance values. The nature of the deviation and the manner in which the deviation is determined will vary depending on the preferred implementation. For example, the deviation could be a standard deviation, a mean square error of fit, or could be assessed as a variance and/or co-variance between the measured impedance values and corresponding points on the curve.

At step 130, the deviation can be used to perform an evaluation of the impedance measurements. In particular, the deviation is assessed to evaluate if the impedance measurement is valid, and in particular whether the measurement is usable or is too inaccurate. In one example, this is achieved by using the deviation to assess the resulting errors in the impedance parameter values, with this being used to assess whether use of the measurement would result in inaccurate parameter values, which could in turn affect resulting indicators. In another example, this is performed using machine learning techniques.

The evaluation can then optionally be used to accept or reject impedance measurements at step 150. This could be performed automatically, but more typically is achieved by displaying results of the evaluation to a user, and allowing the user to make the final decision regarding whether or not a measurement should be rejected. Thus in this instance, the process is performed to assist or guide users in assessing whether or not measurements are accurate and/or usable.

Accordingly, the above described arrangement provides a mechanism to evaluate impedance measurements performed on a biological subject, by comparing deviation of the measurements from a theoretical measurement locus, based on an idealised response of a biological subject. Results of the evaluation can then be used to assist in assessing whether the impedance measurement should be accepted as valid or should be rejected and optionally repeated. Thus, this turns the assessment of measurement validity from being a purely subjective test, to being an at least partially objective assessment, thereby ensuring that less inaccurate measurements are used. This can help ensure that resulting impedance parameter values are more accurate, in turn improving the ability of the system to accurately calculate indicators relating to the biological subject, which can further lead to improved patient outcomes, or the like.

A number of further features will now be described.

As mentioned above, the impedance curve is typically a circular segment. However, it will be appreciated that other curves could be used in some situations. For example, with some scenarios, impedance measurements at higher frequencies can be inaccurate due to capacitive effects. This can be compensated using a phase correction procedure, as will be described in more detail below, which adjusts measured impedance values so that they more correctly align with the circular locus. However, it will be appreciated that as an alternative to performing phase correction, a curve other than a circular locus could be used to account for high frequency effects. Despite this, it will be noted that the use of a circle is particularly advantageous as this represents the idealised biological response and also simplifies the mathematics of evaluating the error, in turn reducing computational requirements for the one or more processing devices.

The evaluation of the impedance measurements is typically used to help determine an impedance measurement validity. In one example, this is achieved by categorising the impedance measurement, for example, providing the measurement with a ranking, such as good, acceptable or bad, which may be denoted by a red, yellow or green, traffic light style indication. However, it will be appreciated that other mechanisms of indicating the likely impedance measurement validity can be used, such as presenting a "confidence of fit" score, such as a %, or the like, which is indicative of a degree of similarity and/or deviation between the impedance curve and the measured impedance values. Such indicators can then be used by an operator, to assist the operator in understanding whether the measurement is acceptable. Additionally and/or alternatively, the assessment could be used to cause the impedance measurement to be repeated automatically. In this instance, no information need be presented to the user, although alternatively a user could be informed the measurement was unacceptable, and asked to check electrode connections or similar. As a further option, the assessment could be used to derive an indicator indicative of the measurement categorisation, or the like, allowing the user to use this in guiding their own assessment of whether the measurement is acceptable.

In one example, the processing devices use the deviation to calculate an estimated error in impedance parameter values derived from the impedance curve and then use the estimated error to evaluate the impedance measurements. In this example, the assessment is performed to take into account that whilst the curve fit might not be particularly accurate, for example if some of the impedance values deviate significantly from the idealised curve, this might nevertheless still result in reasonably accurate parameter values. Accordingly, by estimating the error in the parameter values, this can be used to allow impedance measurements that would be rejected due to deviation alone to be accepted if the deviation does not affect the resulting parameter values. Similarly, this will allow some impedance measurements to be assessed as invalid even if the deviation is minimal, as long as the resulting impedance parameter values would be inaccurate. It will be appreciated that this improves the assessment of measurement validity, taking into account whether inaccuracies would have a material effect on the resulting calculated parameter values.

Whilst a single error could be calculated, more typically the processing device calculates an error associated with each of a number of different impedance parameter values. This allows an assessment to be made of whether relevant parameter values are affected. For example, if the impedance measurements are made for assessing extra-cellular fluid levels only, inaccuracies in $R_{inf}$ might not be problematic, as long as $R_0$ is accurate.

Having calculated one or more estimated errors, the processing devices can compare the estimated error to one or more thresholds and evaluate the impedance measurement based on results of the comparison. In this regard, a threshold can be set to assess whether or not an error in the parameter value is meaningful or not, and hence whether the measurement should be considered as valid.

The thresholds can be of any appropriate form but are typically based on an evaluation of previous impedance measurements. For example, previous measurements can be examined and assessed to determine measurements that should be considered valid, with errors then being calculated and used to define thresholds distinguishing between valid and invalid measurements. The thresholds can be derived from measurements performed on one or more reference subjects, such as reference subjects, using a statistical analysis and/or using machine learning techniques.

It will be appreciated however that generic thresholds might not be particularly accurate. For example, a measurement for an elderly patient having lymphedema may be quite different to a healthy teenager. Accordingly, in one example, thresholds are selected based on characteristics of the current subject, such as a similar age, height, weight, sex, medical symptoms or conditions, ethnicity, or the like. This can then be used to select thresholds derived from reference subjects having similar characteristics, making the thresholds relevant to the current subject.

Additionally and/or alternatively, the thresholds could be selected taking into account an impedance analysis process being performed. For example, as mentioned above, if the impedance measurement process is being performed to determine extra-cellular fluid levels, the value of $R_0$ might need to be accurate, whilst the value of $R_{inf}$ could be significantly less accurate, allowing different thresholds to be used as compared to if intra-cellular fluid levels are being measured.

In another example, subject specific thresholds can be used. In this regard, a custom threshold can be established for individual subjects to take into account that different subjects will respond differently to impedance measurements, so that a measurement that is unacceptable for one subject, might be acceptable for another subject. A good example of this is that subjects with dry skin typically have worse measurements than subjects with normally hydrated skin. In this instance, when a subject first undergoes impedance measurements, a default threshold could be used, with the threshold then being modified to take into account feedback from a user. Thus, the processing device can evaluate the impedance measurement using at least one threshold, determine a user assessment of the impedance measurement in accordance with user input commands and then selectively modify the at least one threshold based on the user assessment. For example, if a measurement is assessed to be unacceptable, but the user chooses to accept the measurement, the threshold could be adjusted to take this into account, allowing the system to become more effective over time.

In one example the processing devices calculate curve coefficients using the curve fitting algorithm and then determine the deviation using the curve coefficients and the impedance values. In one particular example, this is achieved using a method of least squares, with the deviation being determined based on a variance and/or co-variance between the impedance values and corresponding values from the curve.

Having determined the deviation, this is then propagated to the impedance parameter values, allowing an estimated error in each impedance parameter value to be determined.

In one example propagation of the error is achieved by determining the deviation by calculating a co-variance matrix indicative of variances and co-variances associated with the impedance curve and the impedance values and then using the co-variance matrix to estimate the error. In this case, the co-variance matrix is generated based on a mean square error of the impedance curve and the impedance values, for example using the equation:

$$\hat{\sigma}_{\hat{x}}^2 = \hat{\sigma}_{\hat{y}}^2 (A^T A)^{-1}$$

where: A is a matrix of measured reactance values X and resistance values R of the form $A = [1\ R\ X]$ $\hat{\sigma}_{\hat{y}}^2$ is the mean square error of the curve fit

The error can be determined in any appropriate manner and in one example, this is achieved using first order partial derivatives of a vector function, and in particular using a Jacobian transform. In this instance, the estimated error can be determined by applying the Jacobian transform to the co-variance matrix using the equation:

$$\hat{\sigma}_f^2 = J_f\ \hat{\sigma}_{\hat{x}}^2\ J_f^T$$

where: $J$ is a Jacobian transform;

$J^T$ is an inverse Jacobian transform;

$\hat{\sigma}_f^2$ is the error

An error is typically calculated for each of the impedance parameter values, allowing each error value to be compared to a respective threshold so that the evaluation can be performed based on results of the comparison. This is typically performed for at least $R_0$, which is the theoretical impedance at a frequency of 0 kHz and $R_{inf}$, which is the theoretical impedance at an infinite frequency and may also be performed for $R_i$, which is the intracellular impedance.

In this example, the respective equations for calculating the error become:

$$\sigma_{R0} = \sqrt{J_{R0}\ \hat{\sigma}_{\hat{x}}^2\ J_{R0}^T}$$

$$\sigma_{R0}(\%) = \frac{100\ \sigma_{R0}}{R0}$$

$$\sigma_{Rinf} = \sqrt{J_{Rinf}\ \hat{\sigma}_{\hat{x}}^2\ J_{Rinf}^T}$$

$$\sigma_{Rinf}(\%) = \frac{100\ \sigma_{Rinf}}{Rinf}$$

$$\sigma_{Ri} = \sqrt{J_{Ri}\ \hat{\sigma}_{\hat{x}}^2\ J_{Ri}^T}$$

$$\sigma_{Ri}(\%) = \frac{100\ \sigma_{Ri}}{Ri}$$

where: $\boldsymbol{J_{R0}}$ **is** a Jacobian transform for $R_0$;

$\boldsymbol{J^T_{R0}}$ is an inverse Jacobian transform for $R_0$; and,

$\hat{\sigma}^2_{\hat{x}}$ is the co-variance matrix for $R_0$;

$\boldsymbol{J_{Rinf}}$ is a Jacobian transform for $R_{inf}$;

$\boldsymbol{J^T_{Rinf}}$ is an inverse Jacobian transform for $R_{inf}$; and,

$\hat{\sigma}^2_{\hat{x}}$ is the co-variance matrix for $R_{inf}$; and,

$\boldsymbol{J_{Ri}}$ is a Jacobian transform for $R_i$;

$\boldsymbol{J^T_{Ri}}$ is an inverse Jacobian transform for $R_i$; and,

$\hat{\sigma}^2_{\hat{x}}$ is the co-variance matrix for $R_i$.

In some circumstances, measured reactances can have negative values, which is again indicative of an issue with the measurement. Accordingly, in one example, the processing devices can additionally and/or alternatively evaluate the impedance measurement based on a number of negative reactance values.

In one particular example, the processing devices evaluate the impedance measurement as being in a first category if a number of negative reactance measurements exceed a first category reactance threshold or at least one parameter value has an error greater than a respective first category threshold. Similarly the processing device determines an impedance measurement to be in a second category if a number of negative reactance measurements exceed a second category reactance threshold or each parameter value has an error greater than a respective second category threshold. If the measurement is not in the first or second categories, it is then classified in a third category. In this example, the first category corresponds to a bad measurement, the second to a questionable measurement and the third category to an acceptable measurement.

As a further refinement, the processing devices evaluate the impedance measurement as being in the first category if an $R_0$ error is greater than a first category $R_0$ threshold, or in a second category if an $R_i$ error is greater than a first category $R_i$ threshold or an $R_{inf}$ error is greater than a first category $R_{inf}$ threshold.

In the above examples, a deviation is used to determine errors in impedance parameter values, which are then compared to respective thresholds to determine if impedance measurements are acceptable. However, as an alternative approach, the processing devices can evaluate the measurement using at least one computational model embodying a relationship between different deviations and measurement validity. In this instance, the computational model can be obtained by applying machine learning to deviations and assessments of measurement validity obtained from one or more reference subjects.

Thus, machine learning can be used to analyse deviations, and an assessment of whether the deviations are acceptable, with this being used to develop a computational model that can perform assessments based on the deviation directly. Using this approach, the deviation could be assessed in a wide range of manners, and take into account a wide range of different factors, potentially leading to a more accurate assessment.

As mentioned above, a phase correction can be applied to impedance values measured at a frequency higher than a set frequency, with the impedance curve being calculated using the phase corrected impedance values. The manner in which phase correction is performed is generally known and will be described in more detail below.

The above described techniques can be used for impedance measurements used to calculate parameter values, which can then be used to determine a body state value, which could be indicative of a specific condition, or could be a general indication of a measured body parameter value that is in turn indicative of a condition. Examples include, but are not limited to one or more of Body Composition, Dry Lean Mass, Lean Body Mass, Skeletal Muscle Mass, Segmental Lean Analysis, Body Fat Mass, Segmental Fat Analysis, BMI (Body Mass Index), (Percent Body Fat), Visceral Fat Area, Visceral Fat Level, Total Body Water (TBW), Intracellular Water (ICW), Extracellular Water (ECW), ECW/TBW, Segmental Body Water, Segmental ECW/TBW, Segmental ICW Analysis, Segmental ECW Analysis, Body-Fat-LBM Control, BMR (Basal Metabolic Rate), Leg Lean Mass, TBW/LBM, Whole Body Phase Angle, Segmental Phase Angle, Reactance, Impedance of Each Segment per frequency, or Body Water Composition History. The body state could also be indicative of a general level of health or athletic fitness, such as whether the individual is fit or unfit.

In one example, the processing device generates an indicator indicative of the body state value, with the indicator being displayed as part of a representation on a display, such as a client device screen, as will be described in more detail below. Thus, results could be presented as a graphical representation, for example using a pointer and scale to indicate the magnitude and/or changes in the indicator value.

In addition to the above techniques, or as an alternative, evaluation of the impedance measurements can also be performed in accordance with other criteria.

In one example, evaluation can be performed based on the number of impedance values that have a negative reactance value. In this regard, negative reactance values typically result from a poor measurement, for example arising due to poor surface contact between the subject and the electrode. Accordingly, a high percentage of negative reactance values is indicative of a poor measurement.

In this example, the technique typically includes having the one or more processing devices determine a count of impedance values having negative reactance values and then using the count to perform an evaluation of the impedance measurements.

This could be achieved in any appropriate manner, and could include applying a phase correction to impedance values measured at a frequency higher than a set frequency; and then determining the count using the phase corrected impedance values.

In one example, the processing device evaluates the impedance measurement as being in a first category if the count is greater than 25% of a total number of impedance values or in a second category if the count is greater than 14%, although it will be appreciated that these thresholds could be adjusted and are intended to be illustrative only. For example, the first category threshold could be greater than 50%, greater than 40%, greater than 30% or greater than 20%, whereas the second category threshold could be greater than 20%, greater than 15%, greater than 10% or greater than 5%.

In another example, a difference between an impedance parameter value and a measured impedance value could be used to evaluate the impedance measurements. For example, if the difference in values is greater than a certain amount, this could be indicative of an unduly broad variance in the impedance values.

In this example, the processing device typically calculates an impedance parameter value using the impedance values, compares the impedance parameter value to a defined frequency impedance value, the defined frequency impedance value being determined from an impedance value obtained by from impedance measurements performed at a defined measurement frequency and then uses a result of the comparison to perform an evaluation of the impedance measurements.

Different parameter values could be used, in which case different defined frequency impedance value would be used.

For example, the parameter value could $R_0$, which case the defined frequency impedance value is typically an impedance value measured at a low frequency, such as greater than 1 kHz, but less than less than 10 kHz, less than 20 kHz, or less than 30 kHz. In one example, the frequency is between 2 kHz and 4 kHz and is typically about 3 kHz.

In another example, the parameter value is $R_{inf}$ which is the theoretical impedance at an infinite frequency, in which case the defined frequency impedance value is typically an impedance value measured at a high frequency, such as greater than 100 kHz, greater than 500 kHz, less than 10000 kHz, less than 5000 kHz, or less than 2000 kHz. In one example, the defined frequency impedance value is measured at between 500 kHz and 2000 kHz and is typically about 1000 kHz.

As a further alternative, the parameter value is a theoretical impedance at the defined frequency. Thus, in this instance, an impedance measurement performed at a specific frequency (e.g. at 3kHz) can be compared to an impedance parameter value in the form of an impedance value obtained from the fitted curve at the same frequency.

In these examples, the processing device can evaluate the impedance measurement as being in a first category if a magnitude of the difference between the impedance parameter value and the defined frequency impedance value is greater than a first difference threshold, or in a second category if a magnitude of the difference between the impedance parameter value and the defined frequency impedance value is greater than a second difference threshold but below the first difference threshold.

It will be appreciated that these difference thresholds could be adjusted depending on the preferred implementation and/or the level of accuracy required by the particular impedance measurements and the defined frequency and parameter value.

The difference thresholds could be absolute. For example, if the impedance parameter value is $R_0$ the first difference threshold could be 55 Ω, whereas the second difference threshold could be 35 Ω, whereas if the impedance parameter value is $R_{inf}$ the first difference threshold could be 70 Ω, whereas the second difference threshold could be 30 Ω.

However, this is not essential, and alternatively, the difference thresholds could be relative, and may for example be set based on a percentage of the impedance parameter value. Thus, in this example, if the impedance parameter value is $R_0$ the first difference threshold could be 15%, whereas the second difference threshold could be 10%, whereas if the impedance parameter value is $R_{inf}$ the first difference threshold could be 30%, whereas the second difference threshold could be 12%.

Example equations used in calculating the magnitude of the difference are shown below:

$$\left| \overrightarrow{R_0 Z_{3kHz}} \right| = \frac{100 \sqrt{(R_{3kHz} - R_0)^2 + X_{3kHz}{}^2}}{R_0}$$

$$\left|\overrightarrow{R_{inf}Z_{1000kHz}}\right| = \frac{100\sqrt{\left(R_{1000kHz} - R_{inf}\right)^2 + X_{1000kHz}^{\,2}}}{R_{inf}}$$

It will be appreciated from this that a range of different difference thresholds could be used depending on the preferred implementation, the nature of the measurement system and the defined frequency and parameter values.

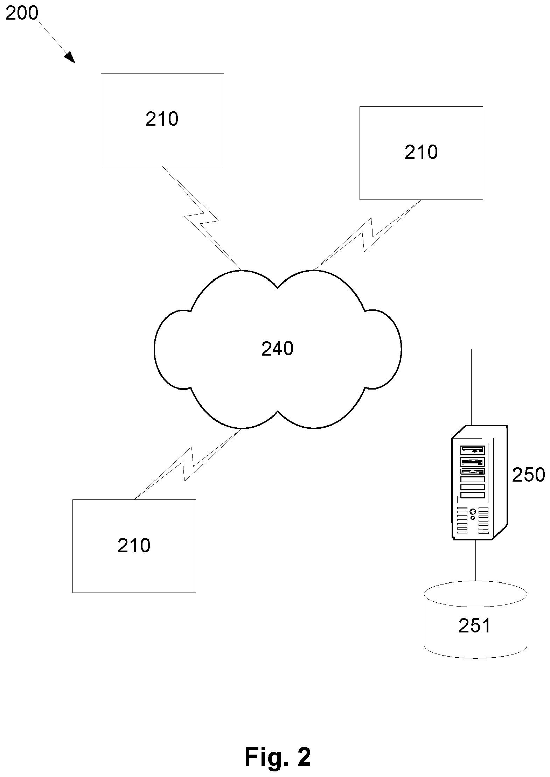

Although not essential, in one example, the above described process is implemented using a distributed architecture including one or more measuring systems in communication with one or more processing devices. An example system will now be described with reference to Figures 2 to 5.

In this example, the system 200 includes a number of measuring systems 210 coupled via a communications network 240 to one or more other measuring systems 210 and/or one or more processing devices, such as a server 250, which may in turn be coupled to a database 251. This arrangement allows data from performed measurements to be collected by the measurement systems 210 and provided to the server 250 for analysis. Collected data may also be stored in the database 251 together with resulting reference signatures and/or heart failure indicators, allowing this information to be remotely accessed and viewed by third parties, such as clinicians, or the like.

In the above arrangement, the communications network 240 can be of any appropriate form, such as the Internet and/or a number of local area networks (LANs) and provides connectivity between the measuring systems 210 and the server 250. It will however be appreciated that this configuration is for the purpose of example only, and in practice the measuring systems 210 and server 250 can communicate via any appropriate mechanism, such as via wired or wireless connections, including, but not limited to mobile networks, private networks, such as an 802.11 network, the Internet, LANs, WANs, or the like, as well as via direct or point-to-point connections, such as Bluetooth, or the like.

It will also be noted that the use of the distributed system is purely optional and the process can be implemented using a standalone measuring system.

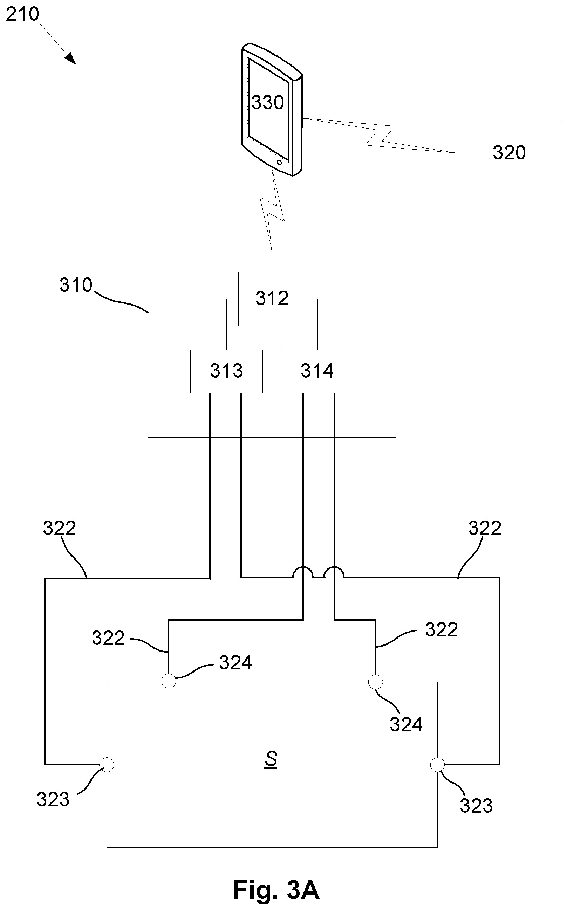

An example measuring system will now be described in further detail with reference to Figure 3A.

In this example, the measuring system includes an impedance measuring unit having an impedance measuring device 310, which is in turn in communication with a processing system in the form of a client device 330, such as a portable computer system, mobile phone, tablet or the like. One or more optional physical characteristic sensors 320 can also be provided for capturing information regarding physical characteristics of an individual/subject.

The nature of the physical characteristic sensors 320 will vary depending on the characteristics to be measured, and could include for example scales for measuring an individual/subject's weight and/or an image capture device, such as a camera, body scanner, DEXA (Dual-Energy X-ray Absorptiometry), 3D laser or optical scanning, or the like, for measuring a height and/or body segment dimensions, as will be described in more detail below. Additionally or alternatively, this could include electronic scales for measuring a weight, and other monitoring equipment, for example for measuring heart rate, blood pressure or other characteristics.

The impedance measuring device 310 typically includes a measuring device processor 312 coupled to at least one signal generator 313 and at least one sensor 314, which are in turn coupled to respective drive and sense electrodes 323 and 324, via leads 322. In use, the signal generator 313 generates a drive signal, which is applied to the individual/subject S via the drive electrodes 323, whilst the sensor 314 measures a response signal via the sense electrodes 324. In use, the measuring device processor 312 controls the at least one signal generator 313 and the at least one sensor 314, allowing the impedance measurements to be performed.

In particular, the measuring device processor 312 is adapted to generate control signals, which cause the signal generator 313 to generate one or more alternating signals, such as voltage or current signals of an appropriate waveform, which can be applied to a subject S, via the first electrodes 323 and processing received signals from the sensor 314. It will be appreciated that the measuring device processor 312 may be any form of electronic processing device capable of performing appropriate control, and could include an FPGA (field programmable gate array), or a combination of a programmed computer system and specialised hardware, or the like.

The signal generator 313 could be of any appropriate form, but will typically include digital to analogue converters (DACs) for converting digital signals from the processing device to analogue signals, which are amplified to generate the required drive signals, whilst the sensor 314 typically includes one or more amplifiers for amplifying sensed response signals and analogue to digital converters (ADCs) to digitise the analogue response signals and providing digitised response signals to the processing device.

The nature of the alternating drive signal will vary depending on the nature of the measuring device and the subsequent analysis being performed. For example, when performing Bioimpedance Spectroscopy (BIS), impedance measurements are performed at each of a number of frequencies ranging from very low frequencies (1 kHz and more typically 3 kHz) to higher frequencies (1000 kHz), and can use as many as 256 or more different frequencies within this range. Such measurements can be performed by applying a signal which is a superposition of plurality of frequencies simultaneously, or a number of alternating signals at different frequencies sequentially, depending on the preferred implementation. The frequency or frequency range of the applied signals may also depend on the analysis being performed.

When impedance measurements are made at multiple frequencies, these can be used to derive one or more impedance parameter values, such as values of R0, Zc, R∞, which correspond to the impedance at zero, characteristic and infinite frequencies. These can in turn be used to determine information regarding both intracellular and extracellular fluid levels, as will be described in more detail below.

A further alternative is for the system to use Multiple Frequency Bioimpedance Analysis (MFBIA) in which multiple signals, each having a respective frequency are injected into the subject S, with the measured impedances being used in the assessment of fluid levels. In one example, four frequencies can be used, with the resulting impedance measurements at each frequency being used to derive impedance parameter values, for example by fitting the measured impedance values to a Cole model, as will be described in more detail below. Alternatively, the impedance measurements at each frequency may be used individually or in combination.

Thus, the measuring device 310 may either apply an alternating signal at a single frequency, at a plurality of frequencies simultaneously, or a number of alternating signals at different frequencies sequentially, depending on the preferred implementation. The frequency or frequency range of the applied signals may also depend on the analysis being performed.

In one example, the applied signal is generated by a voltage generator, which applies an alternating voltage to the subject S, although alternatively current signals may be applied. In one example, the voltage source is typically symmetrically arranged, with two signal generators 313 being independently controllable, to allow the signal voltage across the subject to be varied, for example to minimise a common mode signal and hence substantially eliminate any imbalance as described in copending patent application number WO2009059351.

As the drive signals are applied to the subject, the sensor 314 then determines the response signal in the form of the voltage across or current through the subject S, using second electrodes 324. Thus, a voltage and/or current is measured between the second electrodes 324. In one example, a voltage is measured differentially, meaning that two sensors 314 are used, with each sensor 314 being used to measure the voltage at each second electrode 324 and therefore need only measure half of the voltage as compared to a single ended system. Digitised response signals are then provided to the measuring device processor 312, which determines an indication of the applied drive signal and measured response signals, and optionally uses this information to determine measured impedances.

In the above arrangement, four electrodes are shown, with two forming drive electrodes and two forming sense electrodes. However, this is not essential, and any suitable number of electrodes could be used. Furthermore, a single signal generator and sensor are shown, but again a respective signal generator and sensor could be used for each drive and sense electrode, respectively, and the described arrangement is for the purpose of illustration only.

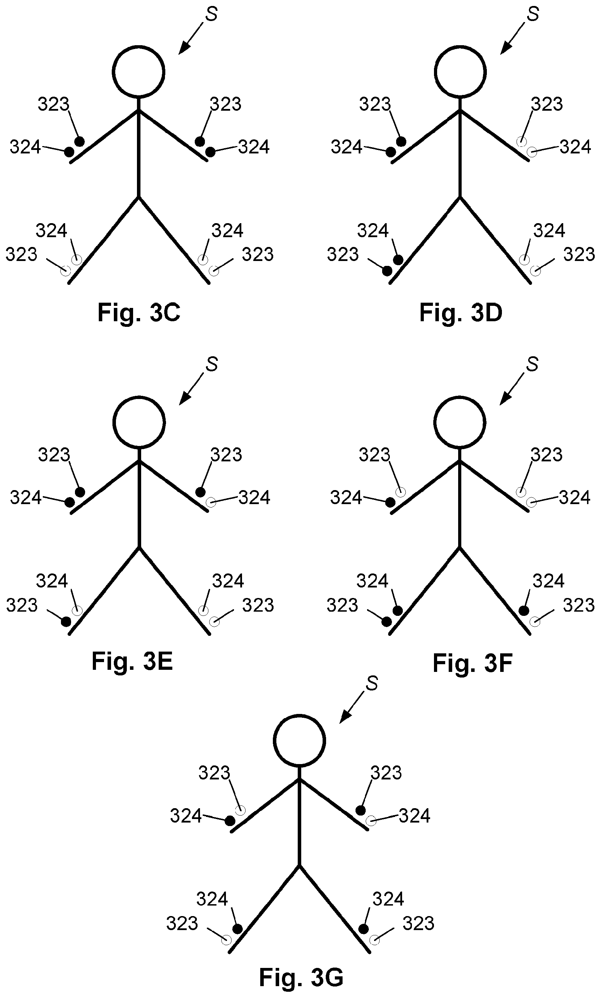

The four electrode arrangement allows a number of different measures to be performed, depending on the configuration of the electrodes. For example, the configuration shown in Figure 3C can then be used to allow torso measurements to be performed, whilst the configurations shown in Figures 3D, 3E, 3F and 3G can be used to allow right whole of body, right arm, right leg and left torso to be measured respectively.

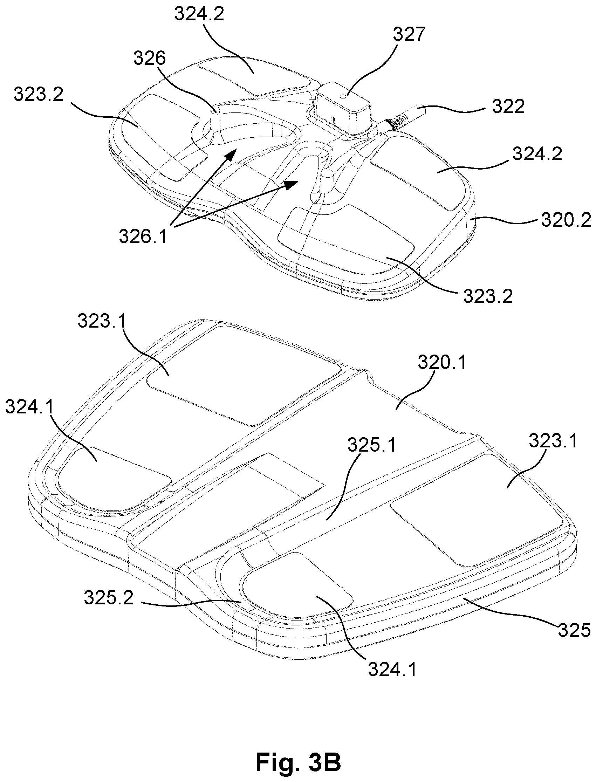

A further example of a physical construction of the measuring device is shown in Figure 3B.

In this example, the measuring device includes first and second housings 320.1, 320.2. The first housing 320.1 has a form factor similar to a set of scales, and includes a generally rectangular body having two spaced pairs of foot drive and sense electrodes 323.1, 324.1 formed from spaced apart metal plates provided on an upper surface, thereby forming footplates on which a user can stand. The second housing 320.2 has a generally rectangular body having two spaced pairs of hand drive and sense electrodes 323.2, 324.2 formed from spaced apart metal plates provided on an upper surface, thereby forming handplates on which a user can rest their hands.

The first housing 320.1 includes a raised section 325, defining a lip 325.1 extending at least partially around each pair of foot drive and sense electrodes to thereby guide positioning of a subject's foot relative to the foot drive and sense electrodes in use. In particular, the raised lip 325.1 includes a rear portion 325.2 configured to engage at least a heel of the user. A similar effect is achieved for the second housing by having a raised portion 326 positioned between each pair of hand drive and sense electrodes, the raised portion defining thumb recesses 326.1 to thereby guide positioning of a subject's thumbs, with the crook of the thumb engaging the raised portion, and hence hands relative to each pair of hand drive and sense electrodes in use.

In this regard, it will be appreciated that whilst this will still allow for some minor variation in positioning between different individuals, for example due to different feet and hand sizes, this helps ensure that any given user's hands and feet are provided at a consistent position relative to the drive and sense electrodes each time the apparatus is used. This provides reproducible positioning, which in turn reduces variations between successive measurements that could be caused by changes in hand or foot position.

This arrangement allows the unit to be used by having the user stand on the first housing, or alternatively sit on a chair, with their feet resting on the foot drive and sense electrodes. The user can then place their hands on the hand drive and sense electrodes on second housing, which can be supported by a desk or table in a seated arrangement, or by a stand or other support for a standing arrangement.

The use of two housing containing separate electrodes, therefore allows impedance measurements to be performed in a variety of circumstances, and in particular allows measurements to be performed in either seated or standing arrangements, which is important in ensuring the system can be used by individuals having restricted physical capabilities. Additionally, the use of metal plate electrodes provided in a housing allows the system to be readily used, and avoids the need for preparation, such as cleaning of tissue surfaces or removal of hair, to allow wet electrodes to be applied to the skin.

It will be appreciated that in this arrangement, electrode configurations similar to those described above with respect to Figures 3C to 3G can be achieved by selectively using respective ones of the drive and sense electrodes 323.1, 324.1, 323.2, 324.2.

In the above arrangement, the client device 330 is in communication with the measuring device processor 312, typically via a wireless communications channels, such as Bluetooth or the like. In one example, the client device 330 is provided in a stand, which is then attached to mounting 327, allowing the client device 330 to be supported on the apparatus in use. This allows the client device 330 to act as a user interface, allowing operation of the impedance measuring device to be controlled, and allowing results of impedance measurements to be displayed.

In particular, the client device 330 can be used to instruct the measuring device processor 312 on a particular sequence of impedance measurements that need to be performed, further receiving either an indication of the drive/sense signals and/or measured impedance values. The client device 330 can then optionally perform further processing, for example to determine the impedance indicators, although alternatively this may not be required and raw impedance values could be provided to the server 250 for analysis.

The client device 330 can also combine impedance values or indicators with information regarding indications of heart failure states and physical characteristics determined either by manual user input or based on signals from one or more physical characteristic sensors. This allows the client device to generate the reference data, which is then transferred via the communications network 240 to the server 250. However, alternatively, the server 250 could obtain the indication of heart failure states and/or physical characteristic from other data sources, depending on the preferred implementation.

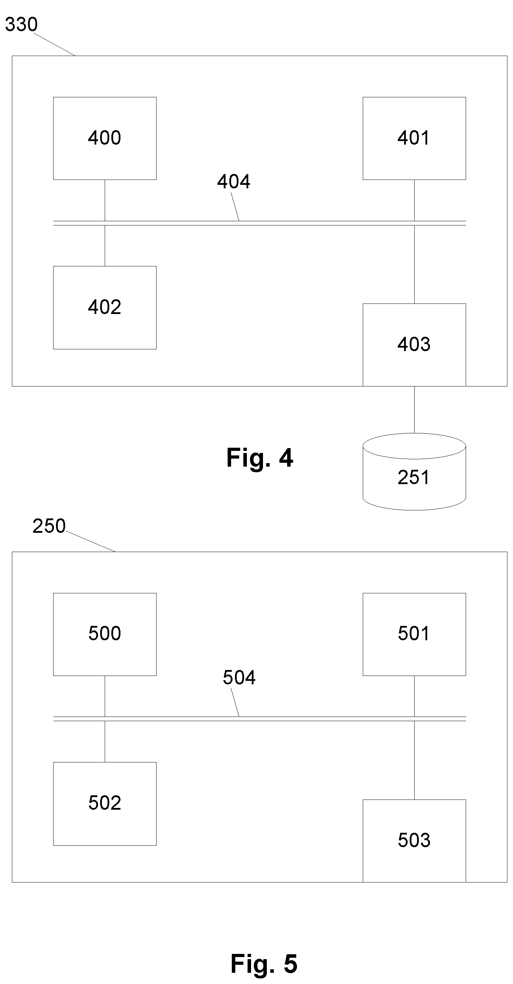

Accordingly, it will be appreciated that the client device 330 can be of any appropriate form and one example is shown in Figure 4. In this example, the client device 330 includes at least one microprocessor 400, a memory 401, an input/output device 402, such as a keyboard and/or display, and an external interface 403, interconnected via a bus 404 as shown. The external interface 403 can be utilised for connecting the client device 330 to peripheral devices, such as the communications networks 240, databases, other storage devices, or the like. Although a single external interface 403 is shown, this is for the purpose of example only, and in practice multiple interfaces using various methods (eg. Ethernet, serial, USB, wireless or the like) may be provided.

In use, the microprocessor 400 executes instructions in the form of applications software stored in the memory 401 to allow communication with the server 250, for example to allow reference data to be provided to the sever, or the like.

Accordingly, it will be appreciated that the client device 330 may be formed from any suitable processing system, such as a suitably programmed PC, Internet terminal, lap-top, or hand-held PC, and in one preferred example is either a tablet, or smart phone, or the like. Thus, in one example, the client device 330 is a standard processing system such as an Intel Architecture based processing system, which executes software applications stored on non-volatile (e.g., hard disk) storage, although this is not essential. However, it will also be understood that the client devices 330 can be any electronic processing device such as a microprocessor, microchip processor, logic gate configuration, firmware optionally associated with implementing logic such as an FPGA (Field Programmable Gate Array), or any other electronic device, system or arrangement.

An example of a suitable server 250 is shown in Figure 5. In this example, the server includes at least one microprocessor 500, a memory 501, an optional input/output device 502, such as a keyboard and/or display, and an external interface 503, interconnected via a bus 504 as shown. In this example the external interface 503 can be utilised for connecting the server 250 to peripheral devices, such as the communications networks 240, databases 251, other storage devices, or the like. Although a single external interface 503 is shown, this is for the purpose of example only, and in practice multiple interfaces using various methods (eg. Ethernet, serial, USB, wireless or the like) may be provided.

In use, the microprocessor 500 executes instructions in the form of applications software stored in the memory 501 to allow the required processes to be performed, including communicating with the client devices 330, and optionally receiving, analysing and/or displaying results of impedance measurements. The applications software may include one or more software modules, and may be executed in a suitable execution environment, such as an operating system environment, or the like.

Accordingly, it will be appreciated that the server 250 may be formed from any suitable processing system, such as a suitably programmed client device, PC, web server, network server, or the like. In one particular example, the server 250 is a standard processing system such as an Intel Architecture based processing system, which executes software applications stored on non-volatile (e.g., hard disk) storage, although this is not essential. However, it will also be understood that the processing system could be any electronic processing device such as a microprocessor, microchip processor, logic gate configuration, firmware optionally associated with implementing logic such as an FPGA (Field Programmable Gate Array), or any other electronic device, system or arrangement. Accordingly, whilst the term server is used, this is for the purpose of example only and is not intended to be limiting.

Whilst the server 250 is a shown as a single entity, it will be appreciated that the server 250 can be distributed over a number of geographically separate locations, for example by using processing systems and/or databases 251 that are provided as part of a cloud based environment. Thus, the above described arrangement is not essential and other suitable configurations could be used.

However, it will be appreciated that the above described configuration assumed for the purpose of the following examples is not essential, and numerous other configurations may be used. It will also be appreciated that the partitioning of functionality between the measuring device 310, client devices 330, and servers 250 may vary, depending on the particular implementation.

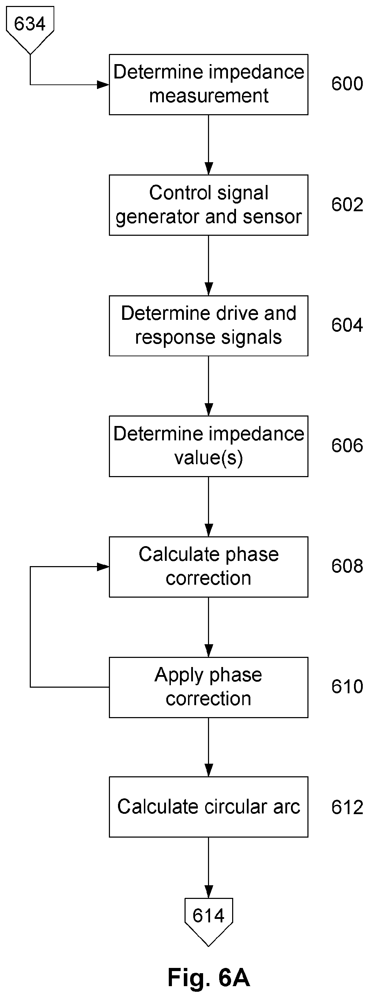

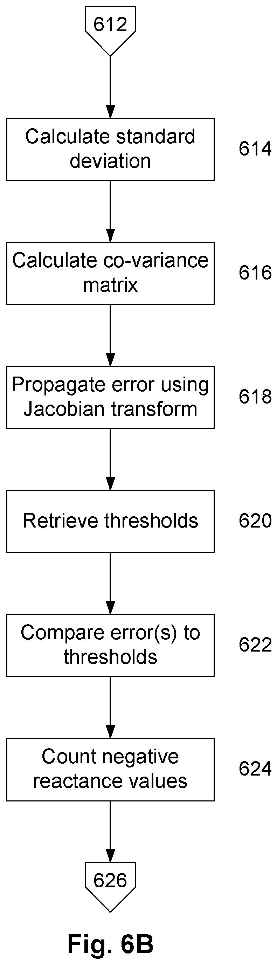

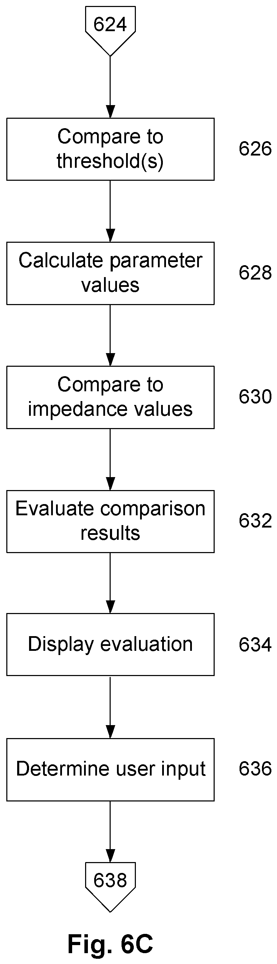

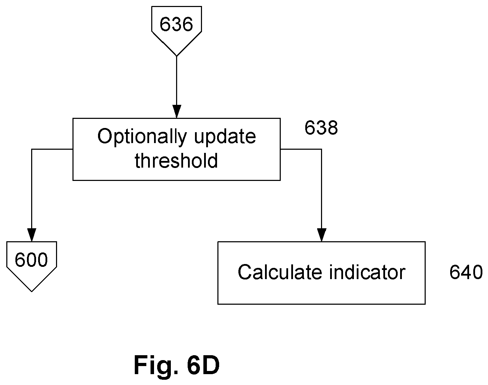

Operation of the system will now be described in further detail with reference to Figures 6A to 6C.

Throughout the following example reference will be made to a user. In this regard, the user will generally be understood to include the subject, but may also encompass an individual assisting the subject in performing the measurement, such as a medical practitioner or the like.

For the purpose of these examples it will also be assumed that a user uses the client device 330 to control the measuring device 310 and optionally any characteristics sensors, allowing impedance measurements to be performed and optionally allowing information regarding physical characteristics to be collected. This is typically achieved by having the user interact with the system via a GUI (Graphical User Interface), or the like presented on the client device 330, which may be generated by a local application, or hosted by the server 250, which is typically part of a cloud based environment, and displayed via a suitable application, such as a browser or the like, executed by the client device 330. Actions performed by the client device 330 are typically performed by the processor 400 in accordance with instructions stored as applications software in the memory 401 and/or input commands received from a user via the I/O device 402. Similarly, actions performed by the server 250 are performed by the processor 500 in accordance with instructions stored as applications software in the memory 501 and/or input commands received from a user via the I/O device 502, or commands received from the client device 330.

In this example, at step 600, the measuring device processor 312 determines the impedance measurement to be performed. This can be achieved in any suitable manner, but would typically include having the user selecting one of a number of available measuring procedures presented on the client device 330, with the client device 330 generating instructions which are provided to the measuring device processor 312.

Prior to a measurement being performed, the first and second electrodes 323, 324 are provided in contact with the subject to allow one or more signals to be injected into the subject S, and allowing a response signal to be measured. The location of the electrodes 323, 324 will depend on the segment of the subject S under study. Thus, for example, the electrodes 323, 324 can be placed on the thoracic and neck region of the subject S to allow the impedance of the chest cavity to be determined. Alternatively, positioning electrodes on the wrist and ankles of a subject allows the impedance of limbs, torso and/or the entire body to be determined. In one example, the general arrangement is to provide electrodes on the hand at the base of the knuckles and between the bony protuberances of the wrist, and on the feet at the base of the toes and at the front of the ankle.

In the arrangement of Figure 3B, prior to the measurement being performed the subject stands on the first housing 320.1, or alternatively sits on a chair, with their feet resting on the foot drive and sense electrodes 323.1, 324.1. The user then places their hands on the hand drive and sense electrodes 323.2, 324.2 on the second housing 320.2, which can be supported by a desk or table in a seated arrangement, or by a stand or other support for a standing arrangement.

At step 602, the measuring device processor 312 controls the signal generator and sensor, causing the drive signals to be applied to the individual/subject and causing the corresponding response signals to be measured, allowing the measuring device processor 312 to determine both the drive and response signals at step 604.

In this regard, the response signal will be a superposition of voltages generated by the human body, such as the ECG (electrocardiogram), voltages generated by the applied signal, and other signals caused by environmental electromagnetic interference. Accordingly, filtering or other suitable analysis may be employed to remove unwanted components.

The acquired signal is typically demodulated to obtain the impedance of the system at the applied frequencies. One suitable method for demodulation of superposed frequencies is to use a Fast Fourier Transform (FFT) algorithm to transform the time domain data to the frequency domain. This is typically used when the applied current signal is a superposition of applied frequencies. Another technique not requiring windowing of the measured signal is a sliding window FFT.

In the event that the applied current signals are formed from a sweep of different frequencies, then it is more typical to use a signal processing technique such as multiplying the measured signal with a reference sine wave and cosine wave derived from the signal generator, or with measured sine and cosine waves, and integrating over a whole number of cycles. This process, known variously as quadrature demodulation or synchronous detection, rejects all uncorrelated or asynchronous signals and significantly reduces random noise.

Other suitable digital and analogue demodulation techniques will be known to persons skilled in the field.

At step 606, the drive and response signals are used to determine impedance values. This can be performed by the measuring device 310 alone, or can be performed in conjunction with the client device 330, for example by transferring measured current and voltage signals to the client device 330, which then analyses these to determine the measured impedances.

In the case of BIS, impedance or admittance measurements are determined from the signals at each frequency by comparing the recorded voltage and the current through the subject. The demodulation algorithm can then produce amplitude and phase signals at each frequency, allowing an impedance value at each frequency to be determined.

The above described process can be performed multiple times, for example to allow impedance measurements to be determined for multiple body segments, including the torso and one or more other segments, such as limbs. Additionally or alternatively, the process may be performed multiple times for the same body segment, with an average (or other statistical measure, such as median, mode, or the like) of two or more measurements being determined and, for example, used for further analysis.

At step 608, a phase correction is typically determined.

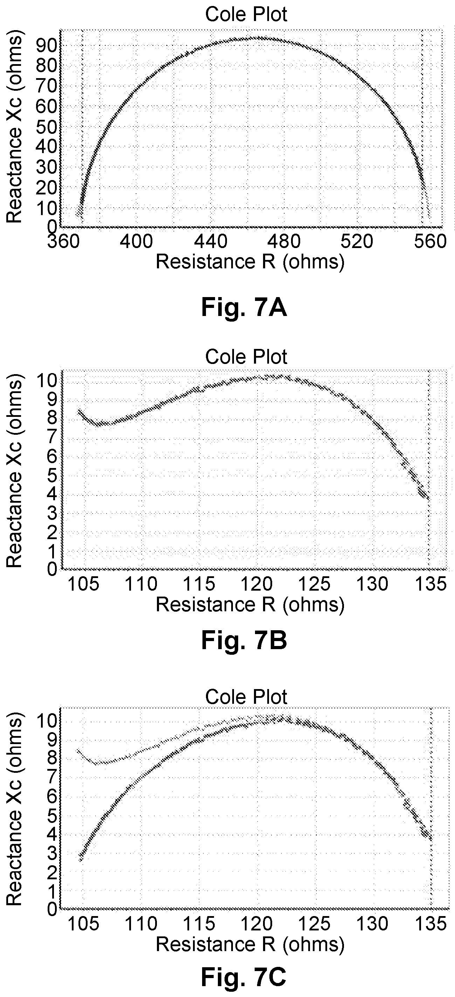

In this regard, BIS measurements on human subjects do not usually show the clean circular arc predicted by the complex impedance model, for a number of reasons including electrical contact issues, subject movement and so on, some of which can be addressed by good measurement procedures. However, one source of error which is difficult to avoid is capacitive leakage, which is especially prevalent at higher frequencies. Because of the physical size of the human body, there is inevitable capacitive coupling between different parts of the body and to the surroundings, which means that some of the current does not pass through the body but bypasses it through the air so that the impedance of the subject in parallel with a capacitor is being measured. The effect on the measurements is generally an increase in reactance at high frequencies, and an example of this is shown in Figures 7A and 7B, which compare a perfect complex impedance plot and a more typical measurement.

Some measurement systems address this by compensation schemes in the analogue circuitry, but if the coupling for any given patient differs from the amount of compensation, there will be a residual increase or decrease in reactance, or sometimes negative reactances. As an alternative, this can be compensated for in software during data analysis, and the most common approach is to apply a phase correction to the raw data, with each measured impedance having a phase correction applied which is proportional to the frequency. This is in turn equivalent to applying a fixed time delay offset to the data set, and so is commonly referred to as a Td correction, although it will be appreciated that a true capacitive shunt model correction would be also an appropriate scheme. The effect of a Td correction is shown in Figure 7C.

The best value of phase correction to apply to the raw data is obtained by using the relationship between the U/V ratio and frequency. Specifically, the value of the phase correction is adjusted iteratively until the relationship is the closest to a straight line. The regression coefficient is used as a measure, and its absolute value should be as close as possible to 1.

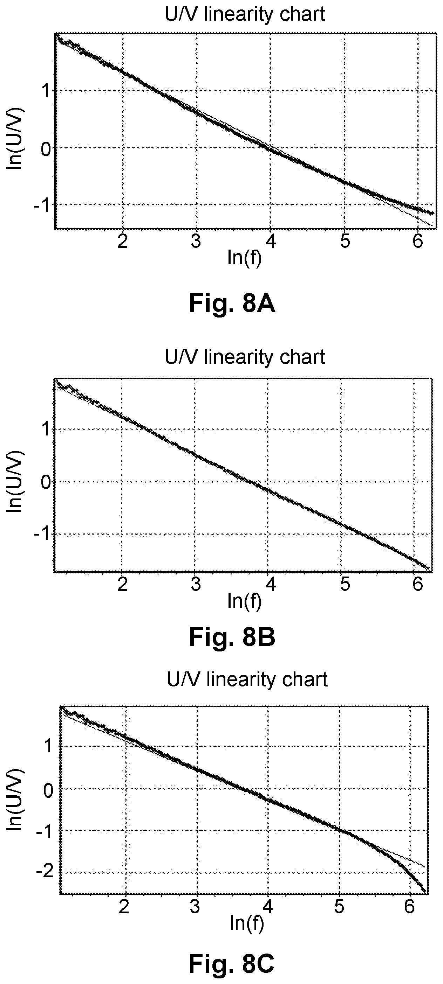

Example U/V plots for the measurement example shown in Figure 7B are shown in Figures 8A to 8C, respectively. Figure 8A shows a U/V plot for the original data, whilst Figure 8B shows optimum phase correction, and Figure 8C showing too much phase correction. Thus, it will be appreciated that the phase correction value can be iteratively determined, until the U/V plot is sufficiently close to a straight line.

In this regard, the process typically includes determining a trial phase correction, applying this to the measured impedance values and calculating the impedance curve. The impedance curve is used to derive the U/V correlation coefficient, with this process being repeated iteratively for different phase correction values until the correlation coefficient reaches a sufficient value.

Since phase correction only affects the high frequency data, and sometimes there may be artefacts at the low frequency part of the spectrum, the optimisation is typically only performed on approximately the upper three quarters of the measurement data (equivalent to frequencies over about 12 kHz).

Once the phase correction is calculated, this is applied to the impedance measurements using the equation:

$$\phi' = \phi - \frac{360.f.T_d}{1,000,000}$$

where: $\phi'$ is the phase after correction, in degrees; and,
$\phi$ is the phase of the measured impedance, in degrees.
$T_d$ is the time delay correction in ns.
$f$ is the measurement frequency in kHz.

After correction corrected values of R' and Xc' are calculated from:

$$R' = |Z| \cos \phi'$$

$$Xc' = |Z| \sin \phi'$$

The corrected data, $\phi'$, R' and $Xc'$ are used in all analysis with the original |Z| instead of $\phi$, R and Xc.

At step 612, the circular arc is calculated for the phase corrected data, which uses an equation of the form:

$$z = mx + py + q$$

where: x, y, and z are variables
m, p and q are coefficients

The equation for a circle with a centre at (a, b) and radius r is:

$$(x - a)^2 + (y - b)^2 = r^2$$

This can be rearranged as:

$$x^2 + y^2 = 2ax + 2by + r^2 - a^2 - b^2$$

Given a set of x and y values this this is solvable by multiple linear regression, as it is of the correct form, where:

$$m = 2a \qquad p = 2b \qquad q = r^2 - a^2 - b^2$$

Substituting resistance, R for x and Reactance X for y, the circle equation becomes:

$$R^2 + X^2 = mR + pX + q$$

Observing that with the impedance values, $Z^2 = R^2 + X^2$, the circle equation can be written as:

$$Z^2 = mR + pX + q$$

The values of m, p and q can be found by multiple linear regression and yield the arc centre and radius, using the regression formulae:

$$p = \frac{\left( \sum_{ZX} \sum_R - \sum_{ZR} \sum_X \right) \left( n \sum_{R^2} - \sum_R^2 \right) - \left( n \sum_{ZR} - \sum_Z \sum_R \right) \left( \sum_{RX} \sum_R - \sum_X \sum_{R^2} \right)}{\left( \sum_R \sum_X - n \sum_{RX} \right) \left( \sum_{RX} \sum_R - \sum_X \sum_{R^2} \right) - \left( \sum_X \sum_{RX} - \sum_{X^2} \sum_R \right) \left( n \sum_{R^2} - \sum_R^2 \right)}$$

$$m = \frac{b \left( \sum_R \sum_X - n \sum_{RX} \right) + \left( n \sum_{ZR} - \sum_Z \sum_R \right)}{n \sum_{R^2} - \sum_R^2}$$

$$q = \frac{\sum_Z - a \sum_R - b \sum_X}{n}$$

Where: n is the number of measured frequencies
$\Sigma_R$ is the sum of all R values for each frequency
$\Sigma_R^2$ is the sum of squares of all R values for each frequency
$\Sigma_X$ is the sum of all X values for each frequency
$\Sigma_X^2$ is the sum of squares of all X values for each frequency
$\Sigma_Z$ is the sum of squares of all Z values for each frequency
$\Sigma_Z^2$ is the sum of squares of squares of all Z values for each frequency
$\Sigma_{RX}$ is the sum of all the products of R and X values for each frequency

$\Sigma_{ZR}$ is the sum of all the products of R and $Z^2$ values for each frequency
$\Sigma_{ZX}$ is the sum of all the products of X and $Z^2$ values for each frequency

The arc centre (a, b) and radius r can be found from m, p and q.

Values for $R_0$, $R_\infty$ and $\alpha$ can then be obtained by simple trigonometry:

$$R_0 = a + \sqrt{r^2 + b^2}$$

$$R_\infty = 2a - R_0$$

$$\alpha = \frac{2}{\pi} arccos\left(\frac{-b}{r}\right)$$

where: arccos result is in radians
if b is positive, there is no valid result for $\alpha$.

In some cases it may be desirable to restrict the frequency range of data used in the curve fitting to avoid unwanted high and low frequency effects. This is controlled by setting maximum and minimum frequencies, between which data is used and outside which data is ignored.

The raw resistance and reactance data is fit to the equation of a circle using multiple linear regression, resulting in the fitted solution for the centre of the circle (a,b) and the radius of the circle (r) as functions of m, p and q.

$$(x - a)^2 + (y - b)^2 = r^2$$

$$(R(f_k) - a)^2 + (X(f_k) - b)^2 = r^2$$

$$R(f_k)^2 - 2aR(f_k) + a^2 + X(f_k)^2 - 2bX(f_k) + b^2 = r^2$$

$$R(f_k)^2 + X(f_k)^2 = r^2 - a^2 - b^2 + 2aR(f_k) + 2bX(f_k)$$

Equivalently:

$$y_k = q + mR_k + pX_k$$

where:

$$y_k = R(f_k)^2 + X(f_k)^2$$

$$R_k = R(f_k)$$

$$X_k = X(f_k)$$

$$q = r^2 - a^2 - b^2$$

$$m = 2a$$

$$p = 2b$$

$$k = 1 \dots F$$

where F = no of frequencies.

$R_0$ and $R_{inf}$ can be calculated from the fitted solution (m,p,q) when reactance equals zero (y = 0).

$$(R_{(0,\infty)} - a)^2 + (0 - b)^2 = r^2$$

$$R_{(0,\infty)}{}^2 - 2aR_{(0,\infty)} + a^2 + (b)^2 = r^2$$

$$R_{(0,\infty)}{}^2 - 2aR_{(0,\infty)} = r^2 - a^2 - b^2$$

$$R_{(0,\infty)}{}^2 - mR_{(0,\infty)} = q$$

$$R_{(0,\infty)}{}^2 - mR_{(0,\infty)} - q = 0$$

Using the solution to a second order polynomial, $R_0$ and $R_{inf}$ are calculated.

$$R_0 = \frac{m + \sqrt{m^2 + 4q}}{2}$$

$$R_\infty = \frac{m - \sqrt{m^2 + 4q}}{2}$$

And thus:

$$R_i = \frac{R_0 R_\infty}{R_0 - R_\infty}$$

$$= \frac{-q}{\sqrt{m^2 + 4q}}$$

The equation of a circle can be rewritten in matrix form:

$$y = Ax$$

where:

$$y = \begin{bmatrix} y_1 \\ y_2 \\ \dots \\ y_F \end{bmatrix}$$

$$A = \begin{bmatrix} 1 & R & X \end{bmatrix}$$

$$x = \begin{bmatrix} q \\ m \\ p \end{bmatrix}$$

At step 614, an estimated error of the solution x = [p,q,r]$^T$ due to the linear least squares fit is calculated, with this being used to estimate a co-variance matrix at step 616, which contains the variances and co-variances associated with each variable, with the variance being equal to the square of the standard deviation. This can then be used to determine the standard deviation of each variable.

The least linear square solution for the previous equation is calculated by:

$$\hat{x} = (A^T A)^{-1} A^T y$$

And the covariance matrix is determined by:

$$\hat{\sigma}_{\hat{x}}^2 = \hat{\sigma}_{\hat{y}}^2 (A^T A)^{-1}$$

Where: $\hat{\sigma}_{\hat{y}}^2$ can be determined by the mean square error of the fit.

At step 618, the error is propagated from one set of variables (m, p, q) to another ($R_0$, $R_{inf}$, $R_i$), which can be calculated using the Jacobian transform along with the covariance matrix as follows.

$$\hat{\sigma}_f^2 = J_f \, \hat{\sigma}_{\hat{x}}^2 \, J_f^T$$

The Jacobian is the matrix of all first order partial derivatives of a vector valued function and is an indication of how one system is converted to another. The Jacobian for this solution is calculated by:

$$J_f = \begin{bmatrix} \dfrac{\partial f}{\partial q} & \dfrac{\partial f}{\partial m} & \dfrac{\partial f}{\partial p} \end{bmatrix}$$

$$J_{R0} = \begin{bmatrix} \dfrac{1}{\sqrt{m^2 + 4q}} & \dfrac{1}{2} + \dfrac{m}{2(\sqrt{m^2 + 4q})} & 0 \end{bmatrix}$$

$$J_{R\infty} = \begin{bmatrix} \dfrac{1}{\sqrt{m^2 + 4q}} & \dfrac{1}{2} - \dfrac{m}{2(\sqrt{m^2 + 4q})} & 0 \end{bmatrix}$$

$$J_{Ri} = \begin{bmatrix} \dfrac{\dfrac{2q}{\sqrt{m^2 + 4q}} - \sqrt{m^2 + 4q}}{\sqrt{m^2 + 4q}} & \dfrac{qm}{^{3/2}\sqrt{\sqrt{m^2 + 4q}}} & 0 \end{bmatrix}$$

The uncertainty in the impedance parameters are then calculated as follows:

$$\sigma_{R0} = \sqrt{J_{R0} \, \hat{\sigma}_{\hat{x}}^2 \, J_{R0}^T}$$

$$\sigma_{R0}(\%) = \dfrac{100 \, \sigma_{R0}}{R0}$$

$$\sigma_{Rinf} = \sqrt{J_{Rinf} \, \hat{\sigma}_{\hat{x}}^2 \, J_{Rinf}^T}$$

$$\sigma_{Rinf}(\%) = \frac{100\ \sigma_{Rinf}}{Rinf}$$

$$\sigma_{Ri} = \sqrt{J_{Ri}\ \hat{\sigma}_{\hat{x}}^2\ J_{Ri}^T}$$

$$\sigma_{Ri}(\%) = \frac{100\ \sigma_{Ri}}{Ri}$$

where: $J_{R0}$ is a Jacobian transform for $R_0$;
$J^T_{R0}$ is an inverse Jacobian transform for $R_0$; and,

$\hat{\sigma}_{\hat{x}}^2$ is the co-variance matrix for $R_0$;
$J_{Rinf}$ is a Jacobian transform for $R_{inf}$;
$J^T_{Rinf}$ is an inverse Jacobian transform for $R_{inf}$; and,

$\hat{\sigma}_{\hat{x}}^2$ is the co-variance matrix for $R_{inf}$; and,
$J_{Ri}$ is a Jacobian transform for $R_i$;
$J^T_{Ri}$ is an inverse Jacobian transform for $R_i$; and,

$\hat{\sigma}_{\hat{x}}^2$ is the co-variance matrix for $R_i$.

At step 620 one or more thresholds can be retrieved. As previously described, the thresholds could be default generic thresholds, or could be customised for the current subject. In one example, the thresholds are set based on physical characteristics of the user, and may take into account the particular impedance measurement process being performed, for example if the impedance measurement forms part of a body composition assessment, as opposed to a lymphedema assessment, or the like.

At step 622, the error(s) calculated as described above are compared to the thresholds to determine if the thresholds are exceeded. Additionally, at step 624, a number of negative reactance values can be calculated, specifically to determine a count of impedance values having a negative reactance value as a proportion of a total number of impedance values, with these being compared to respective thresholds at step 626.

At step 628, impedance parameter values are calculated, with these then being compared to impedance measurements performed at specific frequencies at step 630. In one example, the parameter value corresponds to $R_0$, with this being compared to the phase corrected impedance value determined based on an impedance measured performed at 3 kHz. This is used to calculate a magnitude of the distance between the impedance value and the parameter value, as given by the equation:

$$\left| \overrightarrow{R_0 Z_{3kHz}} \right| = \sqrt{(R_{3kHz} - R0)^2 + X_{3kHz}{}^2}$$

Similarly, the parameter value could be $R_{inf}$ with this being compared to the phase corrected impedance value determined based on an impedance measured performed at 1000 kHz. This is used to calculate a magnitude of the distance between the impedance value and the parameter value, as given by the equation:

$$\left| \overrightarrow{R_{inf} Z_{1000kHz}} \right| = \sqrt{(R_{1000kHz} - Rinf)^2 + X_{1000kHz}{}^2}$$

The results of the comparison are then evaluated at step 632, with this being used to evaluate the measurement, and in one particular example, place the measurement into one of three categories, in this example designated as red, yellow or green, corresponding to measurements that are bad, questionable or acceptable.

An example of the particular assessment criteria are shown below:

- **Red category**

  ◦ Number of negative reactance measurements > red reactance threshold, OR

∘ $\sigma_{R0}(\%)$ > red $R_0$ threshold OR,

$$\circ \quad \left|\overrightarrow{R_0 Z_{3kHz}}\right| > \text{red } R_0 \quad \text{difference threshold}$$

$$\circ \quad \left|\overrightarrow{R_{inf} Z_{1000kHz}}\right| > \text{red } R_{inf} \quad \text{difference threshold}$$

- **Yellow category**

  ∘ Number of **negative** reactance measurements > yellow threshold, OR

  ∘ $\sigma_{R0}(\%)$ > yellow $R_0$ threshold & $\sigma_{Rinf}(\%)$ > yellow $R_{inf}$ threshold & $\sigma_{Ri}(\%)$ > yellow $R_i$ threshold, OR

  ∘ $\sigma_{Ri}(\%)$ > red $R_i$ threshold, OR

  ∘ $\sigma_{Rinf}(\%)$ > red $R_{inf}$ threshold OR

$$\circ \quad \left|\overrightarrow{R_0 Z_{3kHz}}\right| > \text{yellow } R_0 \quad \text{difference threshold}$$

$$\circ \quad \left|\overrightarrow{R_{inf} Z_{1000kHz}}\right| > \text{yellow } R_{inf} \quad \text{difference threshold}$$

It will be appreciated that the actual values of the thresholds will depend on the impedance measuring device used, and that these can be set based on a visual evaluation of example measurements.

At step 634, the evaluation is displayed to a user, allowing the user to make an assessment of whether or not the measurement is to be accepted, which is typically indicated via user input provided via the user interface at step 636.

The feedback can be used to optionally update the thresholds at step 638. For example, if a measurement was categorised as red or yellow, but ultimately accepted by the user, this can be used to update thresholds for the respective subject, so that the categorisation becomes more accurate over time.

If the measurement was not accepted, the process can return to step 600 allowing the measurement to be repeated.

Otherwise the measurements can be used to determine an indicator, such as a body composition indicator or similar at step 640.

In the above example, the errors in the impedance parameter values are used individually in order to categorise the impedance measurement as red, yellow, or green. However, it will be appreciated that other assessments could be made. For example, the errors could be combined to calculate an overall error across the impedance parameter values, with the overall error being used in making the assessment. Such a combination could be performed using a simple summation of errors, or could be calculated in other manners, such as using a weighted sum, overall percentage error, or the like. Additionally, whilst the above example operates by categorizing the measurements, it will be appreciated that other evaluations could be used, such as calculating an overall confidence score, or the like.

The above example also uses multiple linear regressions in order to perform the curve fitting, but will be appreciated that other linear or non-linear approaches can be used, which can in turn simply include calculation of the Jacobian. It will also be appreciated that whilst the above described example has focussed on calculation of the Jacobian matrix for the impedance parameter values $R_0$, $R_{inf}$ and $R_i$, the technique could be extended to other impedance parameter values that can be calculated from the impedance curve, such as $\alpha$, or the like.

The above described approach can be used for a wide range of different parameters, but are particularly suited to measurements of fluid levels, which are often subject to a wide range of variations based on external factors and day to day variations, which are not otherwise necessarily meaningful.

Furthermore, whilst the above examples have focussed on a subject such as a human, it will be appreciated that the measuring device and techniques described above can be used with any animal, including but not limited to, primates, livestock, performance animals, such race horses, or the like.

Throughout this specification and claims which follow, unless the context requires otherwise, the word "comprise", and variations such as "comprises" or "comprising", will be understood to imply the inclusion of a stated integer or group of integers or steps but not the exclusion of any other integer or group of integers. As used herein and unless otherwise stated, the term "approximately" means $\pm 20\%$.

**Claims**

1. An impedance measurement system for performing a bioimpedance measurement on a biological subject, the system including:

a) a signal generator configured to apply alternating signals to at least part of the biological subject at a plurality of different frequencies;
b) a sensor configured to measure response signals from the biological subject; and,
c) one or more electronic processing devices that:

i) determine the bioimpedance measurement as a plurality of impedance values obtained at the plurality of different frequencies using the measured response signals;
ii) calculate an impedance curve using a curve fitting algorithm;
iii) determine a deviation of the impedance curve from the plurality of impedance values of the bioimpedance measurement; and,
iv) use the deviation to perform an evaluation of the bioimpedance measurement **characterised in that** evaluation of the bioimpedance measurement is used to categorise the bioimpedance measurement and derive an indicator indicative of the measurement categorisation.

2. A system according to claim 1, wherein the impedance measurement is categorised as being at least one of:

a) bad;
b) questionable; and,
c) acceptable.

3. A system according to any one of the claims 1 to 2, wherein the one or more processing devices:

a) calculate curve coefficients using the curve fitting algorithm; and,
b) determine the deviation using the curve coefficients and the impedance values.

4. A system according to any one of the claims 1 to 3, wherein the one or more processing devices:

a) use the deviation to calculate an estimated error in impedance parameter values, the impedance parameter values being derived from the impedance curve and the one or more processing devices calculating an estimated error by propagating the deviation to the impedance parameter values; and,
b) use the estimated error to evaluate the impedance measurements.

5. A system according to any one of the claims 1 to 4, wherein the one or more processing devices:

a) determine the deviation by calculating a co-variance matrix indicative of variances and co-variances associated with the impedance curve and the impedance values; and,
b) use the co-variance matrix to estimate the error.

6. A system according to claim 5, wherein at least one of:

a) the co-variance matrix is generated based on a mean square error of the impedance curve and the impedance values.
b) the one or more processing devices calculate the co-variance matrix using the equation:

$$\hat{\sigma}_{\hat{x}}^2 = \hat{\sigma}_{\hat{y}}^2 (\boldsymbol{A}^T \boldsymbol{A})^{-1}$$

where: A is a matrix of measured reactance values X and resistance values R of the *form A = [1 R X]*

$\hat{\sigma}_{\hat{y}}^2$ is the mean square error of the curve fit.

7. A system according to any one of the claims 1 to 6, wherein the one or more processing devices calculate an estimated error at least one of:

   a) using first order partial derivatives of a vector function;
   b) using a Jacobian transform; and,
   c) by applying a Jacobian transform to a co-variance matrix using the equation:

$$\hat{\sigma}_f^2 = J_f \, \hat{\sigma}_{\hat{x}}^2 \, J_f^T$$

   where: $J$ is a Jacobian transform;
   $J^T$ is an inverse Jacobian transform;

   $\hat{\sigma}_f^2$ is the error.

8. A system according to any one of the claims 1 to 7, wherein the one or more processing devices:

   a) calculate an error value associated with a number of impedance parameter values, wherein, optionally:

   i) the impedance parameter values include one or more of:

   (1) $R_0$ which is the theoretical impedance at a frequency of OkHz;
   (2) $R_{inf}$ which is the theoretical impedance at an infinite frequency; and,
   (3) $R_i$ which is the intracellular impedance; and,

   ii) the one or more processing devices calculate error values using the equations:

   (1) for $R_0$:

$$\sigma_{R0} = \sqrt{J_{R0} \, \hat{\sigma}_{\hat{x}}^2 \, J_{R0}^T}$$

$$\sigma_{R0}(\%) = \frac{100 \, \sigma_{R0}}{R0}$$

   where: $J_{R0}$ is a Jacobian transform for $R_0$;
   $J^T{}_{R0}$ is an inverse Jacobian transform for $R_0$; and,

   $\hat{\sigma}_{\hat{x}}^2$ is a co-variance matrix;

   (2) for $R_{inf}$

$$\sigma_{Rinf} = \sqrt{J_{Rinf} \, \hat{\sigma}_{\hat{x}}^2 \, J_{Rinf}^T}$$

$$\sigma_{Rinf}(\%) = \frac{100 \, \sigma_{Rinf}}{Rinf}$$

   where: $J_{Rinf}$ is a Jacobian transform for $R_{inf}$;
   $J^T R_{inf}$ is an inverse Jacobian transform for $R_{inf}$; and,

   $\hat{\sigma}_{\hat{x}}^2$ is a co-variance matrix; and,

(3) for $R_i$

$$\sigma_{Ri} = \sqrt{J_{Ri}\,\hat{\sigma}_{\hat{x}}^2\,J_{Ri}^T}$$

$$\sigma_{Ri}(\%) = \frac{100\,\sigma_{Ri}}{Ri}$$

where: $J_{Ri}$ is a Jacobian transform for $R_i$;
$J^T_{Ri}$ is an inverse Jacobian transform for $R_i$; and,

$\hat{\sigma}_{\hat{x}}^2$ is a co-variance matrix;

b) compare each of error value to at least one respective threshold; and,
c) perform the evaluation based on results of the comparison.

9. A system according to any one of the claims 1 to 8, wherein the one or more processing devices evaluate the impedance measurement based on a number of negative reactance values.

10. A system according to any one of the claims 1 to 9, wherein the one or more processing devices evaluate the impedance measurement as being:

a) in a first category if at least one of:

i) a number of negative reactance measurements exceeds a first category reactance threshold; or
ii) at least one parameter value has an error greater than a respective first category threshold;

b) in a second category if at least one of:

i) a number of negative reactance measurements exceeds a second category reactance threshold;
ii) each parameter value has an error greater than a respective second category threshold;

c) in a third category if it is not in the first or second category.

11. A system according to any one of the claims 1 to 10, wherein the one or more processing devices evaluate the measurement at least in part using one more thresholds, and wherein the thresholds are at least one of:

a) determined based on evaluation of previous impedance measurements performed for the subject;
b) determined based on evaluation of previous measurements impedance performed for one or more reference subjects; and,
c) determined by applying machine learning to evaluation of previous impedance measurements performed for one or more reference subjects.

12. A system according to any one of the claims 1 to 11, wherein the one or more processing devices evaluate the measurement using at least one computational model embodying a relationship between different deviations and measurement validity.

13. A system according to any one of the claims 1 to 12, wherein the one or more processing devices:

a) apply a phase correction to impedance values measured at a frequency higher than a set frequency; and,
b) calculate the impedance curve using the phase corrected impedance values.

14. A system according to any one of the claims 1 to 13, wherein the one or more processing devices:

a) calculate an impedance parameter value using the impedance values;
b) compare the impedance parameter value to a defined frequency impedance value, the defined frequency impedance value being determined from an impedance value obtained from impedance measurements performed at a defined measurement frequency; and,

c) use a result of the comparison to perform an evaluation of the impedance measurements.

15. A method for use in evaluating a bioimpedance measurement performed on a biological subject, the method including, in one or more electronic processing devices:

a) determining the bioimpedance measurement as a plurality of impedance values obtained from impedance measurements performed at a plurality of different frequencies on at least part of the biological subject;

b) calculating an impedance curve using a curve fitting algorithm;

c) determining a deviation of the impedance curve from the plurality of impedance values of the bioimpedance measurement; and,

d) using the deviation to perform an evaluation of the bioimpedance measurement to categorise the bioimpedance measurement and derive an indicator indicative of the measurement categorisation.

## Patentansprüche

1. Impedanzmesssystem zum Durchführen einer Bioimpedanzmessung an einem biologischen Subjekt, wobei das System Folgendes umfasst:

a) einen Signalgenerator, der dazu ausgelegt ist, alternierende Signale bei einer Mehrzahl von unterschiedlichen Frequenzen an zumindest einen Teil des biologischen Subjekts anzulegen;

b) einen Sensor, der dazu ausgelegt ist, Antwortsignale von dem biologischen Subjekt zu messen; und

c) eine oder mehrere elektronische Verarbeitungsvorrichtungen, die:

i) die Bioimpedanzmessung als eine Mehrzahl von Impedanzwerten, die bei der Mehrzahl von unterschiedlichen Frequenzen erhalten werden, unter Verwendung der gemessenen Antwortsignale bestimmen;

ii) eine Impedanzkurve unter Verwendung eines Kurvenanpassungsalgorithmus berechnen;

iii) eine Abweichung der Impedanzkurve von der Mehrzahl von Impedanzwerten der Bioimpedanzmessung bestimmen; und

iv) die Abweichung verwenden, um eine Bewertung der Bioimpedanzmessung durchzuführen, **dadurch gekennzeichnet, dass** die Bewertung der Bioimpedanzmessung verwendet wird, um die Bioimpedanzmessung zu kategorisieren und einen Indikator abzuleiten, der die Messkategorisierung anzeigt.

2. System nach Anspruch 1, wobei die Impedanzmessung als mindestens eines von Folgendem kategorisiert wird:

a) mangelhaft;

b) zweifelhaft; und

c) akzeptabel.

3. System nach einem der Ansprüche 1 bis 2, wobei die eine oder mehreren Verarbeitungsvorrichtungen:

a) Kurvenkoeffizienten unter Verwendung des Kurvenanpassungsalgorithmus berechnen; und

b) die Abweichung unter Verwendung der Kurvenkoeffizienten und der Impedanzwerte bestimmen.

4. System nach einem der Ansprüche 1 bis 3, wobei die eine oder mehreren Verarbeitungsvorrichtungen:

a) die Abweichung verwenden, um einen geschätzten Fehler in Impedanzparameterwerten zu berechnen, wobei die Impedanzparameterwerte aus der Impedanzkurve abgeleitet werden und die eine oder mehreren Verarbeitungsvorrichtungen einen geschätzten Fehler durch Fortpflanzung der Abweichung auf die Impedanzparameterwerte berechnen; und

b) den geschätzten Fehler verwenden, um die Impedanzmessungen zu bewerten.

5. System nach einem der Ansprüche 1 bis 4, wobei die eine oder mehreren Verarbeitungsvorrichtungen:

a) die Abweichung durch Berechnen einer Kovarianzmatrix bestimmen, die Varianzen und Kovarianzen anzeigt, die mit der Impedanzkurve und den Impedanzwerten assoziiert sind; und,

b) die Kovarianzmatrix verwenden, um den Fehler zu schätzen.

**6.** System nach Anspruch 5, wobei mindestens eines von Folgendem zutrifft:

a) die Kovarianzmatrix wird basierend auf einem mittleren quadratischen Fehler der Impedanzkurve und der Impedanzwerte erzeugt;
b) die eine oder mehreren Verarbeitungsvorrichtungen berechnen die Kovarianzmatrix unter Verwendung der folgenden Gleichung:

$$\hat{\sigma}_{\hat{x}}^2 = \hat{\sigma}_{\hat{y}}^2 (A^T A)^{-1}$$

wobei: A eine Matrix von gemessenen Reaktanzwerten X und Widerstandswerten R der Form A = [1 $R$ X] ist

$\hat{\sigma}_{\hat{y}}^2$ der mittlere quadratische Fehler der Kurvenanpassung ist.

**7.** System nach einem der Ansprüche 1 bis 6, wobei die eine oder mehreren Verarbeitungsvorrichtungen einen geschätzten Fehler mit mindestens einem von Folgendem berechnen:

a) unter Verwendung von Teilableitungen erster Ordnung einer Vektorfunktion;
b) unter Verwendung einer Jacobi-Transformation; und
c) durch Anwenden einer Jacobi-Transformation auf eine Kovarianzmatrix unter Verwendung der folgenden Gleichung:

$$\hat{\sigma}_f^2 = J_f \hat{\sigma}_{\hat{x}}^2 J_f^T$$

wobei: J eine Jacobi-Transformation ist;
$J^T$ eine inverse Jacobi-Transformation ist;

$\hat{\sigma}_f^2$ der Fehler ist.

**8.** System nach einem der Ansprüche 1 bis 7, wobei die eine oder mehreren Verarbeitungsvorrichtungen:

a) einen Fehlerwert berechnen, der mit einer Anzahl von Impedanzparameterwerten assoziiert ist, wobei optional:

i) die Impedanzparameterwerte eines oder mehrere von Folgendem umfassen:

(1) $R_0$, das die theoretische Impedanz bei einer Frequenz von 0 kHz ist;
(2) $R_{inf}$, das die theoretische Impedanz bei einer unendlichen Frequenz ist; und
(3) $R_i$, das die intrazelluläre Impedanz ist; und

ii) die eine oder mehreren Verarbeitungsvorrichtungen Fehlerwerte unter Verwendung der folgenden Gleichungen berechnen:

(1) für $R_0$:

$$\sigma_{R0} = \sqrt{J_{R0} \hat{\sigma}_{\hat{x}}^2 J_{R0}^T}$$

$$\sigma_{R0}(\%) = \frac{100\sigma_{R0}}{R0}$$

wobei: $J_{R0}$ eine Jacobi-Transformation für $R_0$ ist;
$J^T_{R0}$ eine inverse Jacobi-Transformation für $R_0$ ist; und

$\hat{\sigma}_{\hat{x}}^2$ eine Kovarianzmatrix ist;

(2) für $R_{inf}$

$$\sigma_{Rinf} = \sqrt{J_{Rinf}\hat{\sigma}_{\hat{x}}^2 J_{Rinf}^T}$$

$$\sigma_{Rinf}(\%) = \frac{100\sigma_{Rinf}}{Rinf}$$

wobei: $J_{Rinf}$ eine Jacobi-Transformation für $R_{inf}$ ist;
$J^T_{Rinf}$ eine inverse Jacobi-Transformation für $R_{inf}$ ist; und

$\hat{\sigma}_{\hat{x}}^2$ eine *Kovarianzmatrix* ist; und

(3) für $R_i$

$$\sigma_{Ri} = \sqrt{J_{Ri}\hat{\sigma}_{\hat{x}}^2 J_{Ri}^T}$$

$$\sigma_{Ri}(\%) = \frac{100\sigma_{Ri}}{Ri}$$

wobei: $J_{Ri}$ eine Jacobi-Transformation für $R_i$ ist;
$J^T_{Ri}$ eine inverse Jacobi-Transformation für $R_i$ ist; und

$\hat{\sigma}_{\hat{x}}^2$ eine Kovarianzmatrix ist;

b) jeden Fehlerwert mit mindestens einem jeweiligen Schwellenwert vergleichen; und
c) die Bewertung basierend auf Ergebnissen des Vergleichs durchführen.

9. System nach einem der Ansprüche 1 bis 8, wobei die eine oder mehreren Verarbeitungsvorrichtungen die Impedanzmessung basierend auf einer Anzahl negativer Reaktanzwerte bewerten.

10. System nach einem der Ansprüche 1 bis 9, wobei die eine oder mehreren Verarbeitungsvorrichtungen die Impedanzmessung als in Folgendem liegend bewerten:

a) in einer ersten Kategorie, wenn mindestens eines von Folgendem zutrifft:

i) eine Anzahl negativer Reaktanzmessungen überschreitet einen Reaktanzschwellenwert der ersten Kategorie; oder
ii) mindestens ein Parameterwert weist einen Fehler auf, der größer als ein jeweiliger erster Kategorieschwellenwert ist;

b) in einer zweiten Kategorie, wenn mindestens eines von Folgendem zutrifft:

i) eine Anzahl negativer Reaktanzmessungen überschreitet einen Reaktanzschwellenwert der zweiten Kategorie;
ii) jeder Parameterwert weist einen Fehler auf, der größer als ein jeweiliger zweiter Kategorieschwellenwert ist;

c) in einer dritten Kategorie, wenn sie nicht in der ersten oder zweiten Kategorie liegt.

**11.** System nach einem der Ansprüche 1 bis 10, wobei die eine oder mehreren Verarbeitungsvorrichtungen die Messung zumindest teilweise unter Verwendung eines mehrerer Schwellenwerte bewerten, und wobei die Schwellenwerte mindestens eines von Folgendem sind:

a) bestimmt basierend auf einer Bewertung vorheriger Impedanzmessungen, die für das Subjekt durchgeführt wurden;

b) bestimmt basierend auf einer Bewertung der Impedanz vorheriger Messungen, die für ein oder mehrere Referenzsubjekte durchgeführt wurden; und

c) bestimmt durch Anwenden von maschinellem Lernen auf die Bewertung vorheriger Impedanzmessungen, die für ein oder mehrere Referenzsubjekte durchgeführt wurden.

**12.** System nach einem der Ansprüche 1 bis 11, wobei die eine oder mehreren Verarbeitungsvorrichtungen die Messung unter Verwendung mindestens eines Rechenmodells bewerten, das eine Beziehung zwischen unterschiedlichen Abweichungen und einer Messvalidität ausdrückt.

**13.** System nach einem der Ansprüche 1 bis 12, wobei die eine oder mehreren Verarbeitungsvorrichtungen:

a) eine Phasenkorrektur auf Impedanzwerte anwenden, die bei einer Frequenz gemessen werden, die höher als eine eingestellte Frequenz ist; und

b) die Impedanzkurve unter Verwendung der phasenkorrigierten Impedanzwerte berechnen.

**14.** System nach einem der Ansprüche 1 bis 13, wobei die eine oder mehreren Verarbeitungsvorrichtungen:

a) einen Impedanzparameterwert unter Verwendung der Impedanzwerte berechnen;

b) den Impedanzparameterwert mit einem definierten Frequenzimpedanzwert vergleichen, wobei der definierte Frequenzimpedanzwert aus einem Impedanzwert bestimmt wird, der aus Impedanzmessungen erhalten wird, die bei einer definierten Messfrequenz durchgeführt werden; und

c) ein Ergebnis des Vergleichs verwenden, um eine Bewertung der Impedanzmessungen durchzuführen.

**15.** Verfahren zur Verwendung beim Bewerten einer Bioimpedanzmessung, die an einem biologischen Subjekt durchgeführt wird, wobei das Verfahren, in einer oder mehreren elektronischen Verarbeitungsvorrichtungen, Folgendes umfasst:

a) Bestimmen der Bioimpedanzmessung als eine Mehrzahl von Impedanzwerten, die aus Impedanzmessungen erhalten werden, die bei einer Mehrzahl von unterschiedlichen Frequenzen an zumindest einem Teil des biologischen Subjekts durchgeführt werden;

b) Berechnen einer Impedanzkurve unter Verwendung eines Kurvenanpassungsalgorithmus;

c) Bestimmen einer Abweichung der Impedanzkurve von der Mehrzahl von Impedanzwerten der Bioimpedanzmessung; und

d) Verwenden der Abweichung, um eine Bewertung der Bioimpedanzmessung durchzuführen, um die Bioimpedanzmessung zu kategorisieren und einen Indikator abzuleiten, der die Messkategorisierung anzeigt.

**Revendications**

**1.** Système de mesure d'impédance destiné à effectuer une mesure de bioimpédance sur un sujet biologique, le système comprenant :

a) un générateur de signaux configuré pour appliquer des signaux alternatifs à au moins une partie du sujet biologique à une pluralité de fréquences différentes ;

b) un capteur configuré pour mesurer les signaux de réponse provenant du sujet biologique ; et

c) un ou plusieurs dispositifs de traitement électronique qui :

i) déterminent la mesure de bioimpédance sous la forme d'une pluralité de valeurs d'impédances obtenues à la pluralité de fréquences différentes à l'aide des signaux de réponse mesurés ;

ii) calculent une courbe d'impédance à l'aide d'un algorithme d'ajustement de courbe ;

iii) déterminent un écart de la courbe d'impédance par rapport à la pluralité de valeurs d'impédances de la mesure de bioimpédance ; et

iv) utilisent l'écart pour effectuer une évaluation de la mesure de bioimpédance, **caractérisé en ce que** l'évaluation de la mesure de bioimpédance est utilisée pour classer la mesure de bioimpédance et dériver un indicateur représentatif de la classification de la mesure.

2. Système selon la revendication 1, dans lequel la mesure d'impédance est classée comme ayant au moins l'une des qualifications suivantes :

   a) mauvaise ;
   b) douteuse ; et
   c) acceptable.

3. Système selon l'une quelconque des revendications 1 et 2, dans lequel les un ou plusieurs dispositifs de traitement :

   a) calculent des coefficients de courbe à l'aide de l'algorithme d'ajustement de courbe ; et
   b) déterminent l'écart à l'aide des coefficients de courbe et des valeurs d'impédances.

4. Système selon l'une quelconque des revendications 1 et 3, dans lequel les un ou plusieurs dispositifs de traitement :

   a) utilisent l'écart pour calculer une erreur estimée dans les valeurs des paramètres d'impédance, les valeurs des paramètres d'impédance étant dérivées de la courbe d'impédance et les un ou plusieurs dispositifs de traitement calculant une erreur estimée en propageant l'écart aux valeurs des paramètres d'impédance ; et
   b) utilisent l'erreur estimée pour évaluer les mesures d'impédances.

5. Système selon l'une quelconque des revendications 1 et 4, dans lequel les un ou plusieurs dispositifs de traitement :

   a) déterminent l'écart en calculant une matrice de covariance indicative des variances et des covariances associées à la courbe d'impédance et aux valeurs d'impédances ; et
   b) utilisent la matrice de covariance pour estimer l'erreur.

6. Système selon la revendication 5, dans lequel au moins l'une des étapes suivantes est exécutée :

   a) la matrice de covariance est générée sur la base d'une erreur quadratique moyenne de la courbe d'impédance et des valeurs d'impédances ;
   b) les un ou plusieurs dispositifs de traitement calculent la matrice de covariance à l'aide de l'équation :

$$\hat{\sigma}_{\hat{x}}^2 = \hat{\sigma}_{\hat{y}}^2 (A^T A)^{-1}$$

où : A est une matrice de valeurs de réactances mesurées X et de valeurs de résistances R de la forme A = [1 R X]

$\hat{\sigma}_{\hat{y}}^2$ est l'erreur quadratique moyenne de l'ajustement de la courbe.

7. Système selon l'une quelconque des revendications 1 à 6, dans lequel les un ou plusieurs dispositifs de traitement calculent une erreur estimée d'au moins une des manières suivantes :

   a) en utilisant des dérivées partielles du premier ordre d'une fonction vectorielle ;
   b) en utilisant une transformation jacobienne ; et
   c) en appliquant une transformation jacobienne à une matrice de covariance à l'aide de l'équation :

$$\hat{\sigma}_f^2 = J_f \hat{\sigma}_{\hat{x}}^2 J_f^T$$

où : $J$ est une transformation jacobienne ;
$J^T$ est une transformation jacobienne inverse ;

$\hat{\sigma}_f^2$ est l'erreur.

8. Système selon l'une quelconque des revendications 1 et 7, dans lequel les un ou plusieurs dispositifs de traitement :

a) calculent une valeur d'erreur associée à un certain nombre de valeurs de paramètres d'impédance, où, en option :

i) les valeurs de paramètres d'impédance comprennent un ou plusieurs des éléments suivants :

$(1)$ $R_0$ qui est l'impédance théorique à une fréquence de 0 kHz ;
$(2)$ $R_{inf}$ qui est l'impédance théorique à une fréquence infinie ; et
$(3)$ $R_i$ qui est l'impédance intracellulaire ; et

ii) les un ou plusieurs dispositifs de traitement calculent des valeurs d'erreurs à l'aide des équations :

$(1)$ pour $R_0$ :

$$\sigma_{R0} = \sqrt{J_{R0}\hat{\sigma}_{\hat{x}}^2 J_{R0}^T}$$

$$\sigma_{R0}(\%) = \frac{100\sigma_{R0}}{R0}$$

où : $J_{R0}$ est une transformation jacobienne pour $R_0$ ;
$J^T_{R0}$ est une transformation jacobienne inverse pour $R_0$ ; et
$\hat{\sigma}_{\hat{x}}^2$ est une matrice de covariance ;

$(2)$ pour $R_{inf}$

$$\sigma_{Rinf} = \sqrt{J_{Rinf}\hat{\sigma}_{\hat{x}}^2 J_{Rinf}^T}$$

$$\sigma_{Rinf}(\%) = \frac{100\sigma_{Rinf}}{Rinf}$$

où : $J_{Rinf}$ est une transformation jacobienne pour $R_{inf}$ ;
$J^T_{Rinf}$ est une transformation jacobienne inverse pour $R_{inf}$ ; et
$\hat{\sigma}_{\hat{x}}^2$ est une matrice de covariance ; et

$(3)$ pour $R_i$

$$\sigma_{Ri} = \sqrt{J_{Ri}\hat{\sigma}_{\hat{x}}^2 J_{Ri}^T}$$

$$\sigma_{Ri}(\%) = \frac{100\sigma_{Ri}}{Ri}$$

où : $J_{Ri}$ est une transformation jacobienne pour $R_i$ ;
$J^T_{Ri}$ est une transformation jacobienne inverse pour $R_i$ ; et
$\hat{\sigma}_{\hat{x}}^2$ est une matrice de covariance ;

b) comparer chacune des valeurs d'erreurs à au moins un seuil respectif ; et

c) effectuer l'évaluation sur la base des résultats de la comparaison.

9. Système selon l'une quelconque des revendications 1 à 8, dans lequel les un ou plusieurs dispositifs de traitement évaluent la mesure d'impédance sur la base d'un nombre de valeurs de réactances négatives.

10. Système selon l'une quelconque des revendications 1 à 9, dans lequel les un ou plusieurs dispositifs de traitement évaluent la mesure d'impédance comme étant :

   a) dans une première catégorie, si au moins l'un des éléments suivants est vrai :

      i) un nombre de mesures de réactances négatives dépasse un seuil de réactance de première catégorie ; ou
      ii) au moins une valeur de paramètre présente une erreur supérieure à un seuil de première catégorie correspondant ;

   b) dans une deuxième catégorie, si au moins l'un des éléments suivants est vrai :

      i) un nombre de mesures de réactances négatives dépasse un seuil de réactance de deuxième catégorie ;
      ii) chaque valeur de paramètre présente une erreur supérieure à un seuil de deuxième catégorie correspondant ;

   c) dans une troisième catégorie, si elle n'appartient pas à la première ni à la deuxième catégorie.

11. Système selon l'une quelconque des revendications 1 à 10, dans lequel les un ou plusieurs dispositifs de traitement évaluent la mesure au moins en partie à l'aide d'un ou plusieurs seuils, et dans lequel les seuils sont au moins l'un des suivants :

   a) déterminés sur la base de l'évaluation de mesures d'impédances précédentes effectuées pour le sujet ;
   b) déterminés sur la base de l'évaluation de mesures d'impédances précédentes effectuées pour un ou plusieurs sujets de référence ; et
   c) déterminés en appliquant un apprentissage automatique à l'évaluation de mesures d'impédances précédentes effectuées pour un ou plusieurs sujets de référence.

12. Système selon l'une quelconque des revendications 1 à 11, dans lequel les un ou plusieurs dispositifs de traitement évaluent la mesure à l'aide d'au moins un modèle de calcul incarnant une relation entre différents écarts et la validité de la mesure.

13. Système selon l'une quelconque des revendications 1 et 12, dans lequel les un ou plusieurs dispositifs de traitement :

   a) appliquent une correction de phase aux valeurs d'impédances mesurées à une fréquence supérieure à une fréquence définie ; et
   b) calculent la courbe d'impédance à l'aide des valeurs d'impédances corrigées en phase.

14. Système selon l'une quelconque des revendications 1 et 13, dans lequel les un ou plusieurs dispositifs de traitement :

   a) calculent une valeur de paramètre d'impédance à l'aide des valeurs d'impédances ;
   b) comparent la valeur de paramètre d'impédance à une valeur d'impédance de fréquence définie, la valeur d'impédance de fréquence définie étant déterminée à partir d'une valeur d'impédance obtenue à partir de mesures d'impédances effectuées à une fréquence de mesure définie ; et
   c) utilisent un résultat de la comparaison pour effectuer une évaluation des mesures d'impédances.

15. Procédé destiné à être utilisé pour évaluer une mesure de bioimpédance effectuée sur un sujet biologique, le procédé comprenant, dans un ou plusieurs dispositifs de traitement électroniques, les étapes suivantes :

   a) déterminer la mesure de bioimpédance sous la forme d'une pluralité de valeurs d'impédances obtenues à partir de mesures d'impédances effectuées à une pluralité de fréquences différentes sur au moins une partie du sujet biologique ;
   b) calculer une courbe d'impédance à l'aide d'un algorithme d'ajustement de courbe ;
   c) déterminer un écart de la courbe d'impédance par rapport à la pluralité de valeurs d'impédances de la mesure de bioimpédance ; et

d) utiliser l'écart pour effectuer une évaluation de la mesure de bioimpédance afin de classer la mesure de bioimpédance et de dériver un indicateur représentatif de la classification de la mesure.

| | |
|---|---|
| Determine impedance values | 100 |

↓

| | |
|---|---|
| Calculate impedance curve | 110 |

↓

| | |
|---|---|
| Determine deviation | 130 |

↓

| | |
|---|---|
| Evaluate impedance measurement | 140 |

↓

| | |
|---|---|
| Optionally accept / reject measurement | 150 |

**Fig. 1**

**Fig. 1B**

**Fig. 1C**

**Fig. 2**

**Fig. 3A**

**Fig. 3B**

Fig. 3C

Fig. 3D

Fig. 3E

Fig. 3F

Fig. 3G

330

**Fig. 4**

251

250

**Fig. 5**

634

Determine impedance
measurement — 600

Control signal
generator and sensor — 602

Determine drive and
response signals — 604

Determine impedance
value(s) — 606

Calculate phase
correction — 608

Apply phase
correction — 610

Calculate circular arc — 612

614

**Fig. 6A**

```
        ┌─────┐
        │ 612 │
        └──┬──┘
           ▼
┌──────────────────────┐
│  Calculate standard  │   614
│      deviation       │
└──────────┬───────────┘
           ▼
┌──────────────────────┐
│ Calculate co-variance│   616
│        matrix        │
└──────────┬───────────┘
           ▼
┌──────────────────────┐
│  Propagate error using│  618
│  Jacobian transform  │
└──────────┬───────────┘
           ▼
┌──────────────────────┐
│  Retrieve thresholds │   620
└──────────┬───────────┘
           ▼
┌──────────────────────┐
│  Compare error(s) to │   622
│      thresholds      │
└──────────┬───────────┘
           ▼
┌──────────────────────┐
│    Count negative    │   624
│  reactance values    │
└──────────┬───────────┘
           ▼
        ┌─────┐
        │ 626 │
        └─────┘
```

## Fig. 6B

```
        ┌─────┐
        │ 624 │
        └──┬──┘
           ▼
```

| | |
|---|---|
| Compare to threshold(s) | 626 |

↓

| | |
|---|---|
| Calculate parameter values | 628 |

↓

| | |
|---|---|
| Compare to impedance values | 630 |

↓

| | |
|---|---|
| Evaluate comparison results | 632 |

↓

| | |
|---|---|
| Display evaluation | 634 |

↓

| | |
|---|---|
| Determine user input | 636 |

↓

```
        ┌─────┐
        │ 638 │
        └─────┘
```

**Fig. 6C**

636

↓

Optionally update threshold    638

600    Calculate indicator    640

## Fig. 6D

Fig. 7A

Fig. 7B

Fig. 7C

U/V linearity chart

Fig. 8A

U/V linearity chart

Fig. 8B

U/V linearity chart

Fig. 8C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170325711 A1
- US 20180184941 A1
- US 20100168530 A1
- US 20100268110 A1
- WO 2009059351 A